# EUROPÄISCHE PATENTSCHRIFT

(11) **EP 0 408 945 B1**
(45) Veröffentlichungstag und Bekanntmachung des Hinweises auf die Patenterteilung: **13.12.1995**
(21) Anmeldenummer: 90112400.8
(22) Anmeldetag: 29.06.1990
(51) Int. Cl.: C12N 15/58, C12N 15/70, C12N 15/67

(54) **Plasmide, deren Herstellung und deren Verwendung bei der Gewinnung eines Plasminogenaktivators**
Plasmides, their preparation and their use in obtaining a plasminogen activator
Plasmides, leur préparation et leur utilisation pour obtenir un activateur de plasminogène

(30) Priorität: 19.07.1989 DE 3923866
(43) Veröffentlichungstag der Anmeldung: 23.01.1991
(73) Patentinhaber: Grünenthal GmbH, D-52078 Aachen (DE)
(72) Erfinder: Brigelius-Flohé, Regina, E., Dr., D-5106 Roetgen (DE); Flohè, Leopold, Prof.Dr., D-5106 Roetgen (DE); Hillen, Wolfgang, Prof.Dr., D-8520 Erlangen (DE); Steffens, Gerd J., Prof.Dr., D-5100 Aachen (DE); Strassburger, Wolfgang, Dr., D-5102 Würselen (DE); Wilhelm, Martin R.F., Dr., D-4000 Düsseldorf (DE)

(56) Entgegenhaltungen:
- EP-A- 0 210 279
- EP-A- 0 236 209
- WO-A-89/03886
- AGRICULTURAL AND BIOLOGICAL CHEMISTRY, Band 52, Nr. 2, Februar 1988, Seiten 329-336, Y. HIBINO et al.: "Enhanced expression of human rpo-urokinase cDNA in Escherichia coli"
- JOURNAL OF BIOCHEMISTRY, Band 101, 1987, Seiten 525-534; T. SATO et al.: "New approaches for the high-level expression of human interleukin-2 cDNA in Escherichia coli"
- NUCLEIC ACIDS RESEARCH, Band 10, Nr. 21, 1982, Seiten 6639-6657; J.D. WINDASS et al.: "The construction of a synthetic Escherichia coli trp promoter and its use in the expression of a synthetic interferon gene"

## Beschreibung

Bei der Therapie von durch Fibrin bedingten Gefäßverschlüssen wie z. B. Herzinfarkt, Lungenembolien, arteriellen Verschlußkrankheiten der Extremitäten usw. spielen Plasminogenaktivatoren eine bedeutende Rolle. Seit etwa Anfang der 50er Jahre ist bekannt, daß in menschlichem Urin ein proteolytisches Enzym enthalten ist, das die Umwandlung von Plasminogen in Plasmin bewirkt, d. h. in das Enzym, welches die Spaltung von Fibrin in lösliche Polypeptide und damit die Auflösung von durch Fibrin bedingten Verschlüssen bewirkt. Dieser Plasminogenaktivator wurde Urokinase genannt, und es zeigte sich, daß tatsächlich verschiedene Urokinaseformen existieren. Alle diese Formen leiten sich aber direkt von dem gleichen Vorläufermolekül, dem seit etwa Mitte der 70er Jahre bekannten Zymogen Prourokinase, ab. Nachdem sich herausgestellt hatte, daß diese Prourokinase eine einkettige Struktur besitzt, wurde sie als scu-PA (single chain urinary plasminogen activator) bezeichnet. Dieser scu-PA besteht aus 411 Aminosäuren und ist in der natürlich vorkommenden Form an der Aminosäure 302 glykosyliert.

Aus verschiedenen Arbeiten ist die gentechnologische Herstellung des unglykosylierten Proteinanteils des scu-PA bekannt, der als "rscu-PA" (rekombinanter scu-PA) bezeichnet wird, oder auch den Freinamen "Saruplase" trägt. Beim therapeutischen Einsatz des rscu-PA hat sich ergeben, daß dieser hinsichtlich Wirkung und Verträglichkeit herkömmlichen Fibrinolytika überlegen ist (vgl. Lancet 1989, Seiten 863 - 868).

Zur Herstellung des unglykosylierten Plasminogenaktivators rscu-PA werden Prokaryonten eingesetzt, wozu auch bereits einige Angaben in der Literatur gemacht wurden. Die bislang bekannten Herstellungsverfahren für den rscu-PA beruhen auf der Expression des aus menschlichen Geweben isolierten Strukturgens des scu-PA. Leider werden dabei nur geringe Expressionsraten erzielt, die eine wirtschaftliche Herstellung des angestrebten Produktes in größerer Menge nicht erlauben. So beschreiben beispielsweise Hibino et al. in Agric. Biol. Chem. 52, 329 - 336 (1988) für eine rscu-PA-Produktion in E.coli eine nur 2 %ige Expressionshöhe, gemessen am Gesamtprotein der Bakterien. Andererseits werden in der europäischen Patentanmeldung 92 182 A2 sowie von Holmes et al. (10) die erreichten Expressionsraten nicht genau angegeben. Bei der Nacharbeitung dieser Angaben wurden jedoch in verschiedenen Stämmen weniger als 2 % des Bakterienproteins an rscu-PA erhalten.

Es sei darauf hingewiesen, daß, wie in den meisten Fällen der Expression eukaryotischer Proteine in Bakterien auch der rscu-PA in der Zelle als denaturierter Einschlußkörper - der hierin nachstehend als "Vorstufenprotein" bezeichnet wird - anfällt, welcher nach intermediärer Isolierung proteinchemisch zur richtigen Tertiärstruktur des rscu-PA zurückgefaltet werden muß. Das so gewonnene Produkt kann dann zur Bestimmung der gebildeten rscu-PA-Menge z. B. durch Zugabe von Plasmin in zweikettige unglykosylierte Urokinase ("rtcu-PA") überführt werden, deren enzymatische Aktivität ermittelt wird und als Maß für die gebildete Menge an rscu-PA dienen kann. Natürlich kann man aber die Ausbeute an Vorstufenprotein bzw. rscu-PA und damit auch die Expressionsrate z. B. mit densitometrischer Auswertung der gelelektrophoretischen Trennung der Gesamtproteine ermitteln.

Es wurde nun überraschend gefunden, daß mit den erfindungsgemäßen Plasmiden transformierte Bakterien eine um ein Vielfaches höhere Expressionsrate als die mit aus dem Stand der Technik bekannten Plasmide transformierten identischen Wirtsstämme erbrachten. Die erfindungsgemäßen Plasmide sind dementsprechend besser als alle bisher bekannten Plasmide zur Gewinnung von rscu-PA bzw. dessen Vorstufenprotein geeignet. Der Vollständigkeit halber sei noch erwähnt, daß in an sich bekannter Weise mit Hilfe der bereits oben für analytische Zwecke erwähnten Spaltung des rscu-PA, beispielsweise mit Plasmin, auch der rtcu-PA in technischem Maßstab erhalten werden kann, nachdem aufgrund der vorliegenden Erfindung der rscu-PA gut zugänglich wurde.

Die erfindungsgemäßen Plasmide zeichnen sich weiterhin dadurch aus, daß ihr Operon einen regulierbaren, gegebenenfalls synthetischen Promotor, eine als Ribosomenbindestelle wirksame Shine-Dalgarno-Sequenz, ein Startcodon, ein synthetisches Strukturgen für scu-PA und stromabwärts vom Strukturgen mindestens einen Terminator aufweist. Vorzugsweise liegen zwei aufeinander folgende Terminatoren vor, bei denen es sich insbesondere um den trp A Terminator und/oder den tet A/orf L Terminator aus Tn 10, vergleiche Schollmeier et al. (6), handelt.

Bei dem regulierbaren Promotor handelt es sich insbesondere um den Trp-Promotor oder den Tac-Promotor.

In der Kontrollregion der erfindungsgemäßen Plasmide beträgt der Abstand zwischen der Shine-Dalgarno-Sequenz und dem Startcodon 6 - 12, vorzugsweise 8 - 10 Nukleotide.

Bei der Konstruktion des in die erfindungsgemäßen Plasmide einzusetzenden Strukturgens werden vorzugsweise die für die jeweiligen Aminosäuren codierenden in der folgenden Tabelle aufgeführten Basensequenzen eingebaut. Dabei müssen in dem Strukturgen nicht alle diese Merkmale gleichzeitig erfüllt sein.

| Aminosäure | Triplett |
|---|---|
| Arginin | CGT |
| Leucin | CTG |
| Valin | GTT |
| Prolin | CCG |
| Glycin | GGT |

Andererseits ist es beim Aufbau des Strukturgens zweckmäßig, die Verwendung folgender Codons für die jeweils genannten Aminosäuren soweit wie möglich zu vermeiden (wobei wiederum diese Merkmale nicht für alle Aminosäuren gleichzeitig erfüllt sein müssen):

| zu vermeidendes Codon | Aminosäure |
|---|---|
| ATA | Isoleucin |
| GTC | Valin |
| CCC | Prolin |
| AAG | Lysin |
| AGG, AGA, CGG, CGA | Arginin |
| GGA, GGG | Glycin |

Durch die erfindungsgemäße Auswahl der Codons für die einzelnen Aminosäuren im Strukturgen sowie die vorstehend genannten Merkmale der Kontrollregion wird die Ausbildung stabiler Sekundärstrukturen zwischen dem Strukturgen und der Kontrollregion weitgehend verhindert.

Zur Gewinnung des synthetischen Strukturgens werden zunächst Oligonukleotide in Abschnitten von 40 - 80 Basen einzelsträngig synthetisiert. Zweckmäßig werden diese im 1»Mol-Maßstab nach der Festphasenmethode von Adams et al. (7) mit Hilfe eines DNA-Synthesizers (Modell Biosearch 8600 der Firma New Brunswick Scientific Co.) hergestellt. Als monomere Synthone finden dabei die handelsüblichen β-Cyanoethyl-geschützten Diisopropylamino-Phosphoramidite der jeweils benötigten Desoxyribonukleoside Anwendung. Nach Abspaltung vom Träger werden die endständig noch tritylgeschützten Stränge unter sterilen Bedingungen durch Gelfiltration entsalzt und vorgereinigt und dann in zwei Läufen einer ersten Trennung durch "reversed phase"-HPLC unterworfen. Das jeweilige Hauptprodukt wird detrityliert und ergibt nach zwei weiteren "reversed phase"-HPLC-Reinigungsschritten das gewünschte Oligonukleotid in hoher Reinheit, wie die gelelektrophoretische Analyse (PAGE) zeigt.

Aus Gruppen von 4 bis 9 dieser Einzelstränge werden dann ca. 200 Nukleotide lange doppelsträngige Fragmente dadurch gewonnen, daß man die nicht-endständigen einzelsträngigen Oligonukleotide am 5'-Ende phosphoryliert und die jeweiligen Komplementärstränge zusammen mit den endständigen Oligonukleotiden annealt und ligiert. Nach Ligation werden die gebildeten klonierfähigen doppelsträngigen Fragmente dann in geeignete linearisierte Vektoren eingesetzt. Die weitere Vorgehensweise ergibt sich aus den weiter unten folgenden Beispielen.

Die in der vorliegenden Anmeldung benutzten Abkürzungen haben die folgenden Bedeutungen:
- bp:: Basenpaare
- DE 52:: Anionenaustauscher der Firma Whatman
- EDTA:: Ethylendiamintetraessigsäure
- IPTG:: Isopropyl-β-D-thiogalaktopyranosid
- PAGE:: Polyacrylamidgelelektrophorese
- PU:: Ploug units
- SDS:: Natriumdodecylsulfat
- S.D.-Sequenz:: Shine-Dalgarno-Sequenz
- Tris-HCl:: Trishydroxymethylaminomethan-Hydrochlorid
- Tween-80:: Polyethylenoxyd(20)sorbitanmonooleat

Zur Konstruktion der erfindungsgemäßen Plasmide geht man vorzugsweise vom handelsüblichen Plasmid pBR 322 (4363 bp) aus. Vorzugsweise wird aus diesem die nic/bom-Region und/oder das Tetracyclinresistenzgen eliminiert. Dabei ist es nicht erforderlich, das Tetracyclinresistenzgen vollkommen zu entfernen, vielmehr ist es hinreichend, wenn dies nur insoweit geschieht, daß das Plasmid den damit transformierten Bakterienstämmen keine Tetracyclin-Resistenz mehr verleiht. Durch diese Maßnahmen (Entfernen der nic/bom-Region und zumindest eines Teils des Tetracyclinresistenzgens) erhält man ein sogenanntes "Hochsicherheitsplasmid".

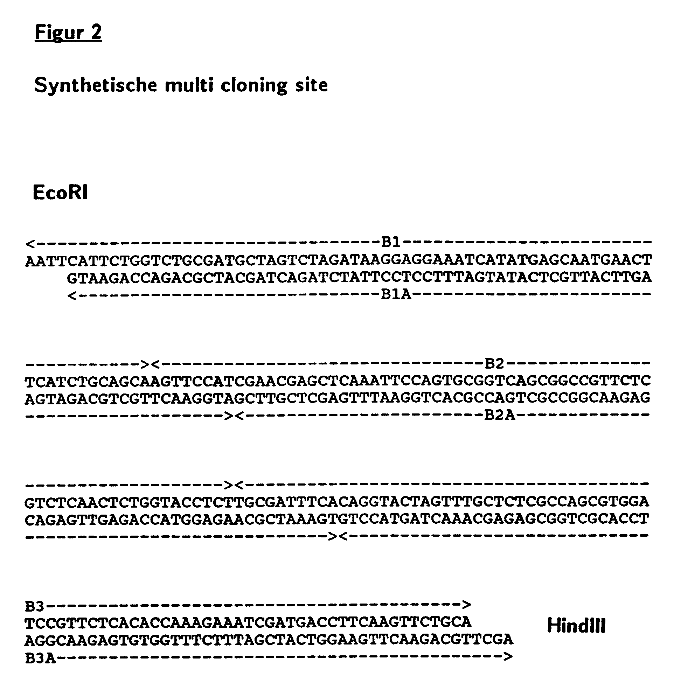

Die bevorzugte Strategie zur Konstruktion eines erfindungsgemäßen Plasmids, ausgehend von dem wie vorstehend ausgeführt modifizierten Plasmid pBR 322 (oder einem anderen der hierin genannten vorbekannten Plasmide) sieht als nächsten Schritt den Einbau einer synthetischen "Multi cloning site" zwischen den Schnittstellen Eco RI und Hind III vor. Diese Multi cloning site (vgl. Figur 2) weist eine festgelegte Reihenfolge von Schnittstellen auf, die dazwischen liegenden Basen sind willkürlich gewählt mit Ausnahme der Sequenz zwischen Xba I und Nde I, die die Ribosomenbindestelle (Shine-Dalgarno-Sequenz) aus dem B. subtilis Xyl Operon 5′-AAGGAG-3′(4) sowie einen festgelegten Abstand zwischen der S.D.-Sequenz und dem Startcodon ATG enthält. Die auf die S.D.-Sequenz folgenden Basen GAAAT sind aus (4) übernommen und mit CATATG, einer Nde I Schnittstelle verbunden. Somit beträgt der Abstand zwischen der S.D.-Sequenz und ATG 8 Basenpaare. Die Abstände zwischen den einzelnen Schnittstellen der Multi cloning site sollten aus kloniertechnischen Gründen mindestens ca 20 Nukleotide lang sein, wodurch das Entfernen der Zwischenfragmente erleichtert wird.

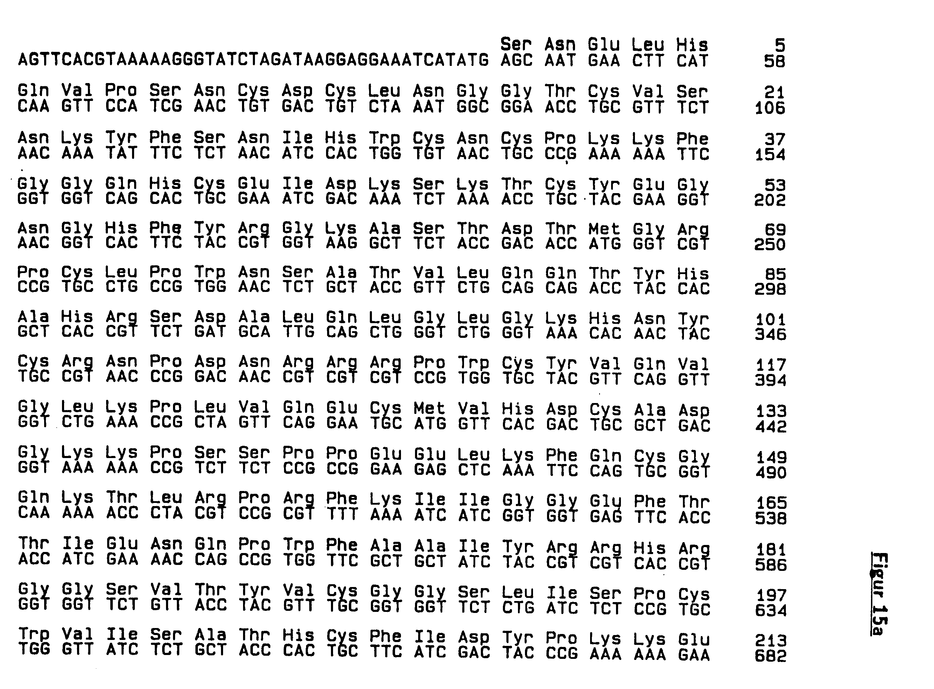

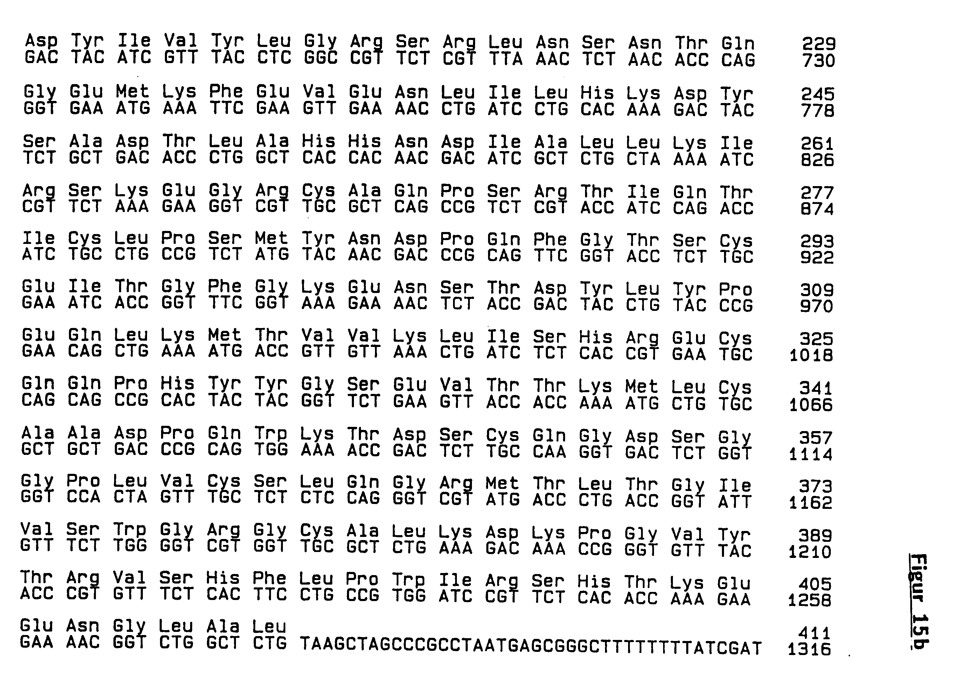

Mit Hilfe dieser Multi cloning site werden in das Plasmid Schnittstellen eingeführt, die - zweckmäßig nach Einbau eines Transkriptionsterminators - den nacheinander erfolgenden Einbau von Teilsequenzen des scuPA-Strukturgens, vorzugsweise beginnend vom C-Terminus des angestrebten Proteins, also vom 3′-Terminus des DNA-Stranges des herzustellenden Strukturgens aus, sowie den Einbau z. B. eines synthetischen oder auch eines aus einem anderen Plasmid isolierten oder handelsüblichen Trp-Promotors ermöglichen. Die vollständige Nukleotidsequenz des so erhaltenen scu-PA-Strukturgens ist mit Angabe der den einzelnen Codons entsprechenden Aminosäuren in Figur 15 dargestellt.

Andere der erfindungsgemäßen Plasmide lassen sich aus einem wie vorstehend beschrieben konstruierten Plasmid z. B. dadurch erhalten, daß man durch Schnitte mit Eco RI und Hind III das synthetische scuPA-Strukturgen mit allen Regulationseinheiten gewinnt und dann in ein anderes, in Enterobacteriaceen, insbesondere in E.coli autonom vermehrungsfähiges Plasmid einbaut, das dazu durch Schnitte mit Eco RI und Hind III linearisiert und verkürzt wurde. In im Prinzip gleicher Weise, aber unter Ausnutzung der Schnittstellen Eco RI und Xba I läßt sich auch z. B. der Trp-Promotor gegen den Tac-Promotor (und umgekehrt) in einem der erfindungsgemäßen Plasmide austauschen.

In analoger Weise kann man auch von anderen bekannten handelsüblichen Plasmiden ausgehen, die singuläre Eco RI- und Hind III-Schnittstellen besitzen, wie z. B. pUC 9, pUC 12, pUC 13, pUC 18, pUC 19 und andere.

Erfindungsgemäße Plasmide mit einem verlängerten Abstand zwischen der Shine-Dalgarno-Sequenz und dem Startcodon ATG lassen sich dadurch erhalten, daß man ein wie oben beschrieben konstruiertes Plasmid, in dem der erwähnte Abstand z. B. 8 Nukleotide beträgt, mit Nde I schneidet, die resultierenden Schnittstellen auffüllt und anschließend die entstehenden blunt-Enden ligiert, wodurch ein erfindungsgemäßes Plasmid gebildet wird, in dem der Abstand zwischen S.D.-Sequenz und dem Startcodon z. B. 10 Nukleotide beträgt.

Wie bereits gesagt, erbringen mit den erfindungsgemäßen Plasmiden transformierte Bakterien eine um ein Vielfaches höhere Expressionsrate als die bei Transformation mit den aus dem Stand der Technik bekannten Plasmiden zu erreichenden. Die Transformation geeigneter kompetenter Wirtsorganismen erfolgt in an sich bekannter Weise. Für die Expression der erfindungsgemäßen Plasmide besonders geeignete Wirtsorganismen sind Stämme der Art E.coli und verwandter Enterobakteriaceen, wie z. B. Stämme von Pseudomonas, Salmonella, Enterobacter, Klebsiella oder Serratia.

Bevorzugte Wirtsorganismen sind Stämme der Art E.coli wie z. B. E.coli GRT-1 und insbesondere E.coli-Stämme der Untergruppe K12 wie z. B. E.coli K12 JM101 (ATCC 33876), E.coli K12 JM103 (ATCC 39403), E.coli K12 JM105 (DSM 4162), der E.coli-K12-Stamm ATCC 31446 oder auch E.coli K12 DH1 (ATCC 33849).

Die Expressionseinleitung in E.coli-Expressionssystemen, die den Tac-Promoter enthalten, kann z. B. durch Lactosezugabe oder Glucoseentzug, vorzugsweise aber durch Zugabe von Isopropyl-β-D-thiogalactopyranosid bewirkt werden. Liegt jedoch in dem Plasmid der Trp-Promoter vor, so erfolgt die Induktion vorzugsweise mit Indolacryl-, -essig- oder -propionsäure. Andere bekannte Induktoren können selbstverständlich in gleicher Weise benutzt werden.

Nach der Induktion und Erreichen einer bestimmten vorgegebenen Zelldichte werden die Zellen abzentrifugiert und der Zentrifugationsrückstand nach Aufschlämmen in einer wässrigen Salzlösung z. B. in einen Homogenisator überführt, in dem die Zellen durch Druckdifferenzen zum Platzen gebracht werden. Nach erneutem Zentrifugieren erhält man im Rückstand das Vorstufenprotein des rscu-PA neben wasserunlöslichen Zelltrümmern etc.. Durch Behandlung mit Guanidinhydrochloridlösung geht das Vorstufenprotein in Lösung, und nach erneutem Zentrifugieren wird die überstehende Lösung mit einem Redoxsystem (beispielsweise reduziertes und oxydiertes Glutathion enthaltend) behandelt, wodurch die Ausbildung der natürlichen Konformation, d. h. die Bildung des angestrebten rscu-PA bewirkt wird. Dieser liegt dann im Reaktionsgemisch in gelöster Form vor und kann durch übliche Reinigungsschritte (z. B. Chromatographie und anschließende Gefriertrocknung) in reiner Form isoliert werden.

Für analytische Zwecke geht man zweckmäßigerweise so vor, daß man die mit dem zu untersuchenden Plasmid transformierten E.coli-Stämme fermentiert und nach Erreichen einer vorgegebenen optischen Dichte der Zellsuspension mit einem geeigneten Induktor die Expression des rscu-PA-Vorstufenproteins induziert. Nach entsprechender Fermentationsdauer werden aliquote Teile entnommen und die daraus abzentrifugierten Zellen dann mit Lysozym (1 mg Lysozym pro ml 50mM Trishydrochloridpuffer mit dem pH 8,0, 50 mM EDTA und 15 % Saccharose) aufgeschlossen. Das Homogenat der lysierten Zellen wird in 4 - 5 M Guanidiniumhydrochloridlösung solubilisiert und nach Verdünnen auf 1,2 M Guanidiniumhydrochlorid unter Zusatz eines Reduktionsmittels (Glutathion, Mercaptoethanol oder Cystein) für 2 - 5 Stunden der Rückfaltungsreaktion unterworfen (vgl. auch z. B. Winkler et al. in (11)). Der so erhaltene einkettige rscu-PA wird durch Zugabe von Plasmin in zweikettigen rtcu-PA umgewandelt, dessen Aktivität dann mit dem Substrat S2444 (pyroGlu-Gly-Arg-p-nitroanilid; Kabi Diagnostika, Schweden), das nur von zweikettigen aktiven Urokinasen gespalten wird, bestimmt wird. Diese Aktivierung des rscu-PA mit Plasmin erfolgt in 50 mM Tris-Puffer, 12 mM Natriumchlorid, 0,02 % Tween 80 bei pH 7,4 und 37° C. Das Verhältnis rscu-PA zu Plasmin sollte etwa 100 bis 1500 zu 1, bezogen auf Molarität, oder etwa 8.000 bis 36.000 zu 1, bezogen auf Enzymeinheiten, betragen. Die Testinkubation erfolgt in 50 mM Tris-Puffer, 38 mM Natriumchlorid bei pH 8,8 in Gegenwart von 0,36 »M Aprotinin (zur Hemmung des Plasmins) und 0,27 mM S 2444 bei 37° C. Je nach dem Gehalt der Probe an rscu-PA wird die Reaktion nach 5 - 60minütiger Inkubation durch Zusatz von 50 %iger Essigsäure gestoppt und die Extinktion bei 405 nm gemessen. Nach den Angaben des Herstellers des Substrats S 2444 bedeutet bei dieser Vorgehensweise eine Extinktionsänderung von 0,05 pro Minute bei 405 nm eine Aktivität von 25 Ploug-Einheiten/ml Testlösung.

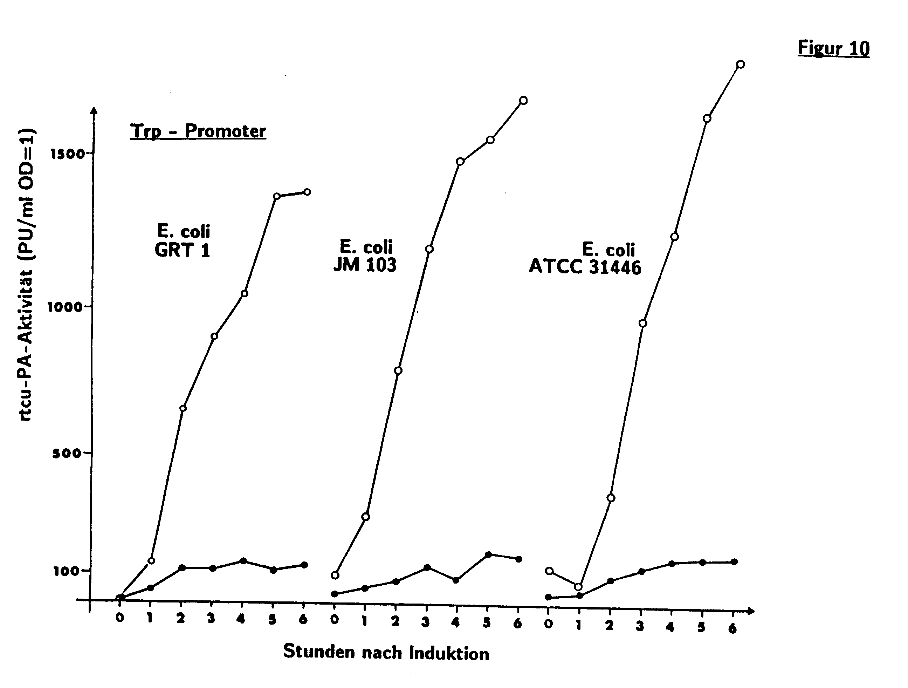

Die Ausbeuten an rscu-PA, die aus unter Verwendung verschiedener erfindungsgemäßer Plasmide in verschiedenen Bakterien gewonnenem Vorstufenprotein erhalten wurden, sind in den Figuren 10, 12 und 14 wiedergegeben.

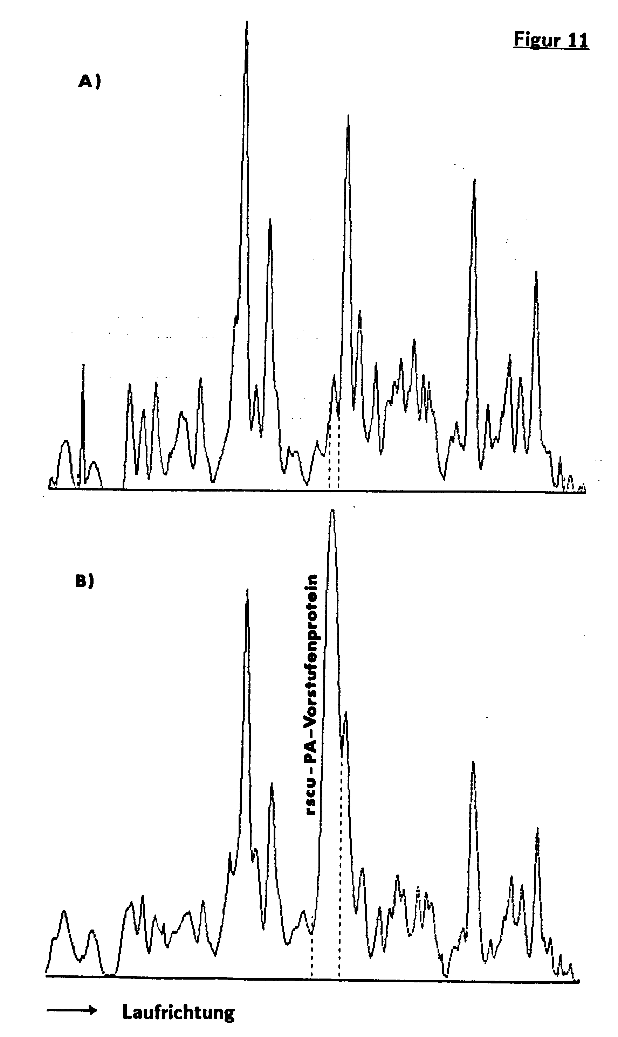

Wie sich aus Figur 11 sowie den Angaben der weiter unten folgenden Beispiele - insbesondere Tabelle 1 aus Beispiel 2 - ergibt, bewirken die erfindungsgemäßen Plasmide vorzugsweise in Stämmen von E.coli die Bildung des rscu-PA-Vorstufenproteins mit einer Expressionsrate von 10 bis 25 Gewichtsprozent, insbesondere 14 bis 20 Gewichtsprozent des gebildeten Gesamtproteins. Die Expressionsrate ist also bei Verwendung der erfindungsgemäßen Plasmide wenigstens 10 bis 15 mal höher als die mit bekannten Plasmiden, wie z. B. pUK 54 trp 207-1 erreichbare.

Die anliegenden Figuren zeigen:
Figuren 1a und 1b: Konstruktion des Plasmids pBF 158 aus dem handelsüblichen Plasmid pBR 322. Dabei werden nacheinander die nic/bom-Region und ein großer Teil des Tetracyclinresistenzgens entfernt.
Figur 2: Die Nukleotidsequenz der synthetischen "Multi cloning site".
Figur 3: Konstruktion des Plasmids pBF 158-01. Dargestellt ist der Einbau der synthetischen Multi cloning site in das Plasmid pBF 158 zwischen die Schnittstellen Eco RI und Hind III.
Figur 4: Konstruktion des Plasmids pBF 158-01 T. Das Fragment Cla I x Hind III wird durch das entsprechende Fragment "T" aus dem Plasmid pRT 61 (5), auf dem sich der tet A/orf L Terminator aus Tn 10 (6) befindet, ersetzt.
Figuren 5a bis 5g: Nukleotidsequenzen der synthetischen Fragmente für das für humane scu-PA codierende Gen (deren Einbau unter Ausbildung des Strukturgens für scu-PA in einem erfindungsgemäßen Plasmid, ausgehend von dem Plasmid pBF 158-01 T in den Beispielen 1d bis 1f beschrieben und in Figuren 6, 7 und 9 dargestellt wird).
Figuren 6a bis 6c: Konstruktion der Plasmide pBF 158-02 T bis pBF 158-06 T durch Einbau der Oligonukleotidfragmente M 4 bis M 8, vom C-Terminus des angestrebten Proteins (entsprechend dem 3'-Terminus des DNA-Stranges) aus, beginnend mit dem Plasmid pBF 158-01 T.
Figuren 7a bis 7c: Konstruktion des Plasmids pBF 158-08 T über eine Hilfskonstruktion im Plasmid pUC 19 (vgl. Beispiel 1e).
Figur 8: Nukleotidsequenz des synthetischen Trp-Promotors.
Figur 9: Konstruktion des Expressionsplasmids für das Vorstufenprotein des humanen rscu-PA, pBF 160. Das Strukturgen für den scu-PA ist durch den schwarzen Balken zwischen den Schnittstellen Nde I und Cla I dargestellt.
Figur 10: Expressionsraten des Vorstufenproteins des humanen rscu-PA (bestimmt als rtcu-PA-Aktivität nach Rückfalten und Aktivierung) in verschiedenen mit dem Plasmid pBF 160 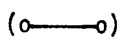 oder mit dem Plasmid pUK 54 trp 207-1 (10) 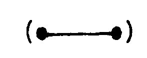 transformierten E.coli-Stämmen unter Kontrolle des Trp-Promotors in Abhängigkeit von der Zeit nach Induktion mit Indolacrylsäure zum Zeitpunkt 0. Angegeben sind die mit dem Substrat S 2444 ermittelten rtcuPA-Aktivitäten in Ploug-Units pro ml eines Fermentationsmediums mit der optischen Dichte gleich 1 bei 578 nm.
Figur 11: Densitogramm einer SDS-PAGE von Proteinen aus mit pBF 161 transformierten E.coli K 12 JM 103-Zellen nach Fermentation vor (A) und 6 Stunden nach (B) Zugabe von Indolacrylsäure als Induktor.
Figur 12: Expressionsraten des Vorstufenproteins des humanen rscu-PA (bestimmt als rtcu-PA-Aktivität nach Rückfalten und Aktivierung) in E.coli K12 JM103 transformiert mit pBF 171, d. h. unter Kontrolle des Tac-Promotors. Die Induktion erfolgte zum Zeitpunkt 0 mit IPTG. Angegeben ist die ermittelte rtcu-PA-Aktivität gegenüber dem Substrat S 2444 in Ploug-Units pro ml eines Fermentationsmediums mit der optischen Dichte gleich 1 bei 578 nm.
Figur 13: Verlängerung des Abstandes zwischen der Shine-Dalgarno-Sequenz (SD) und dem Startkodon ATG von 8 auf 10 Basen durch Auffüllen der Nde I-Schnittstellen und anschließende Ligation der entstehenden blunt-Enden.
Figur 14: Expressionsraten des Vorstufenproteins des humanen rscu-PA (bestimmt als rtcu-PA-Aktivität nach Rückfalten und Aktivierung) unter Kontrolle des Trp-Promotors in E.coli GRT-1 transformiert mit dem Plasmid pBF 161 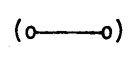 oder pBF 163 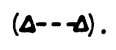 Die Induktion erfolgte mit Indolacrylsäure unmittelbar nach dem Zeitpunkt 0. Angegeben sind die mit dem Substrat S 2444 ermittelten rtcuPA-Aktivitäten in Ploug-Units pro ml eines Fermentationsmediums mit der optischen Dichte gleich 1 bei 578 nm.
Figuren 15a und 15b: Die vollständige Nukleotidsequenz des scu-PA-Strukturgens mit Angabe der den einzelnen Codons entsprechenden Aminosäuren.

Die in den Ausführungsbeispielen verwendeten Restriktionsenzyme sind handelsüblich (vgl. z. B. Nachr. Chem. Techn. Lab. 35, 939, 1987).

Das in den Beispielen zur Selektion der Klone benutzte Kulturmedium enthält pro Liter: 7,8 g Pepton aus Fleisch, 7,8 g Pepton aus Casein, 10 g Hefeextrakt, 5,6 g Natriumchlorid und 10 g Glucose. Dieses Medium wird mit 10 g Agar pro Liter in der Wärme versetzt und in Platten ausgegossen.

### Beispiel 1

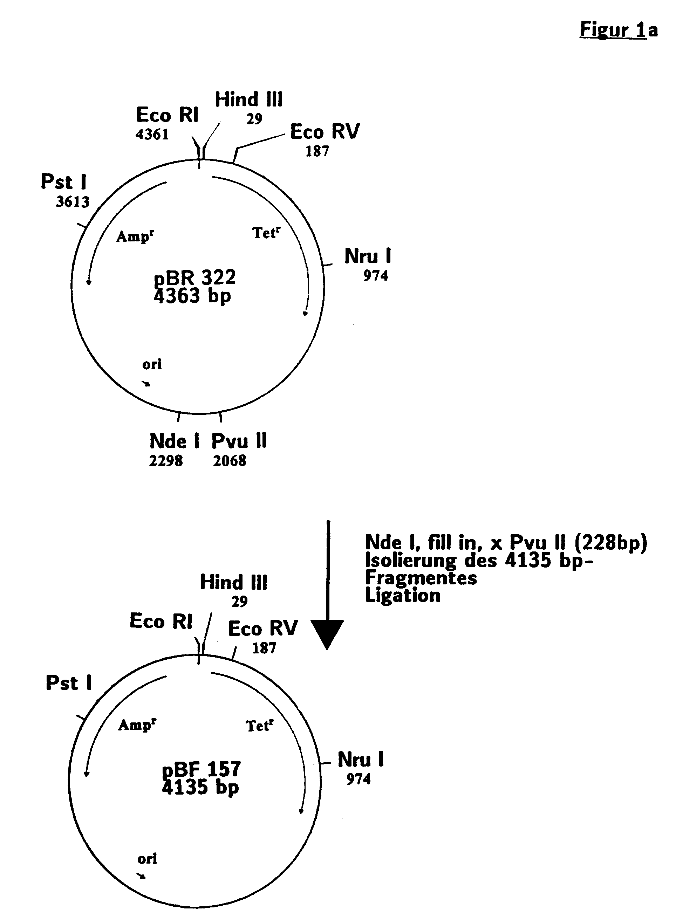

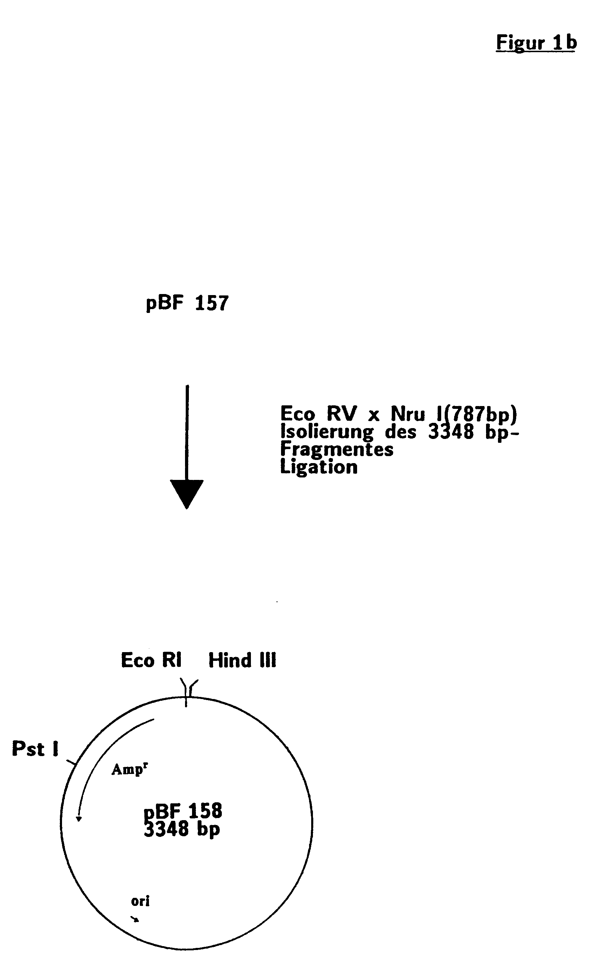

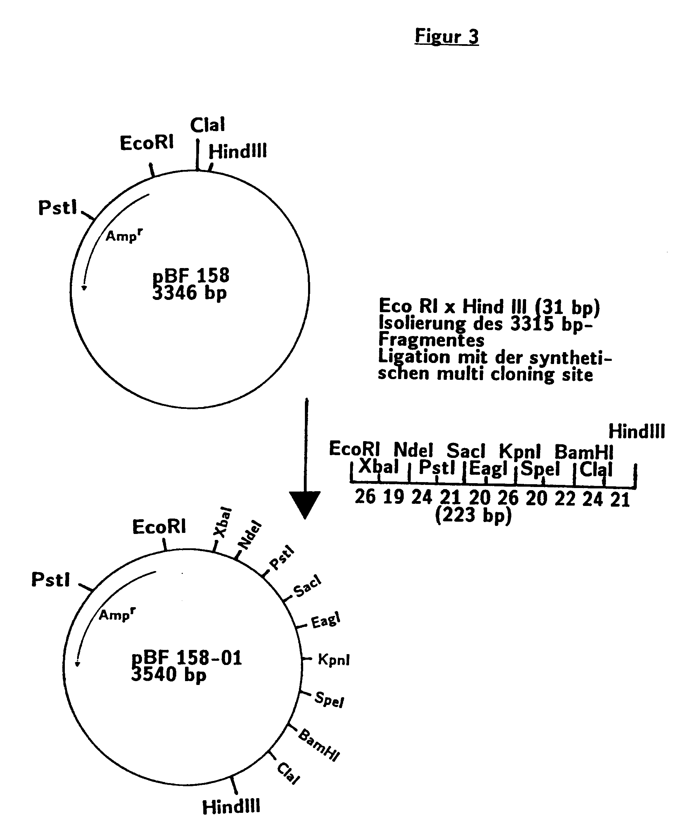

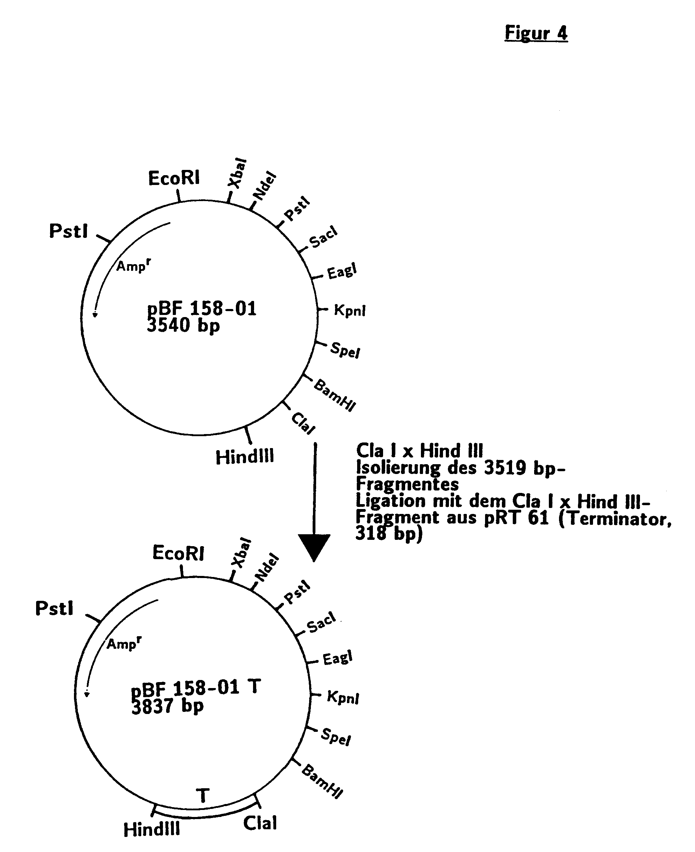

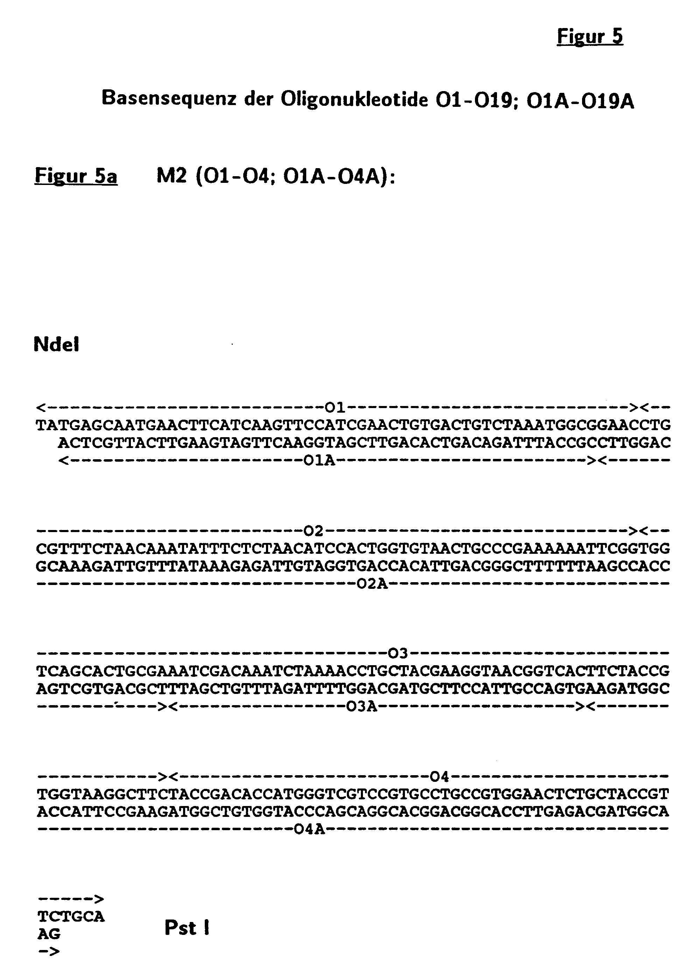

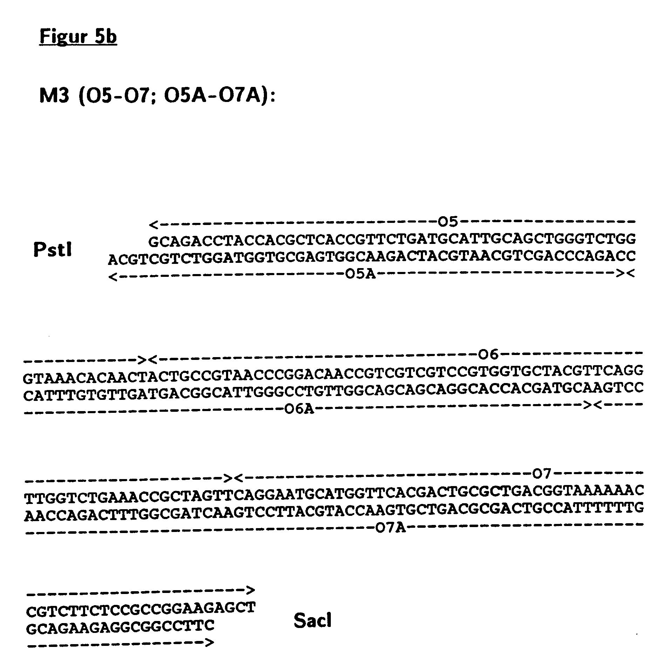

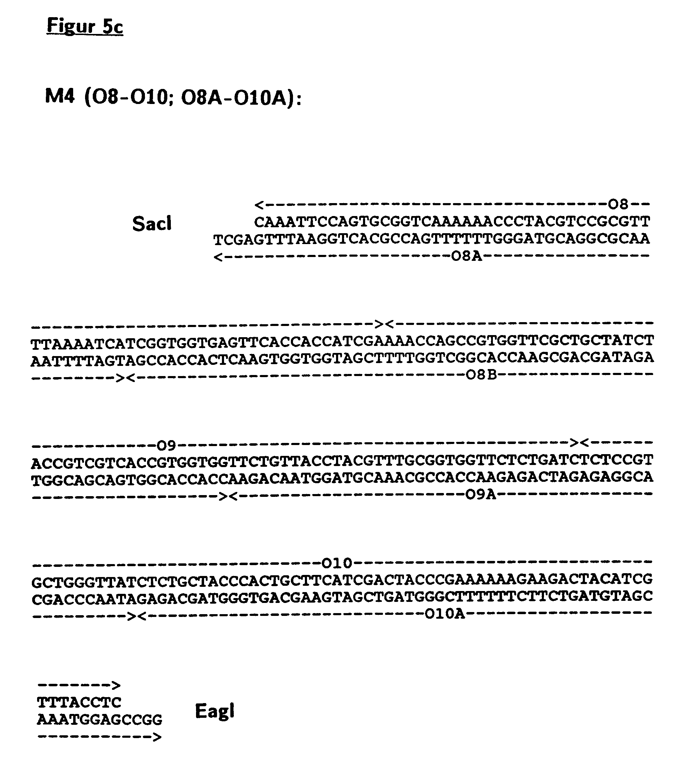

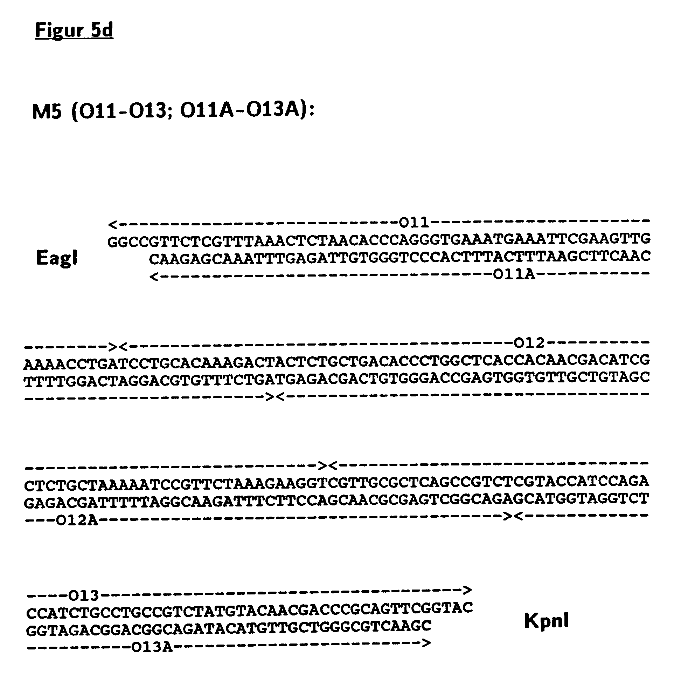

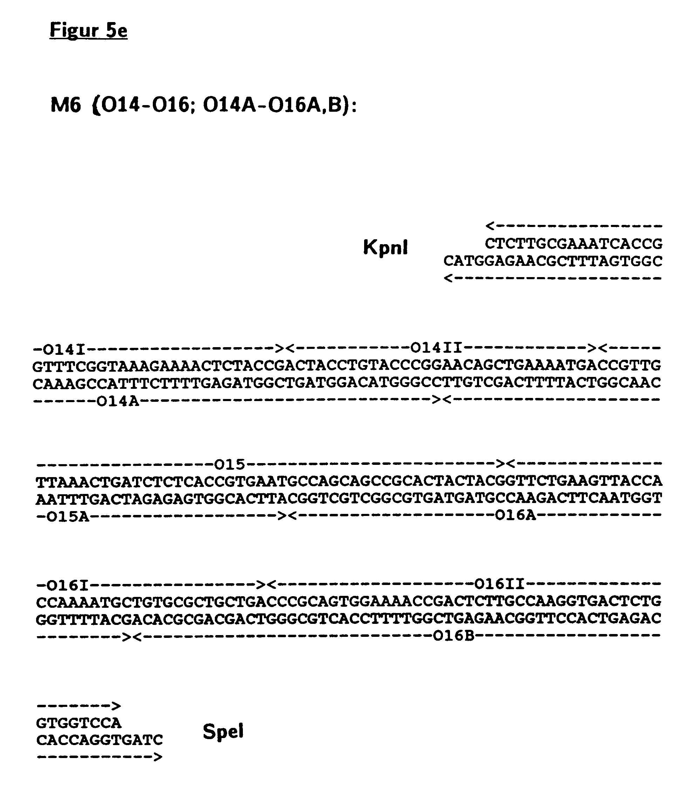

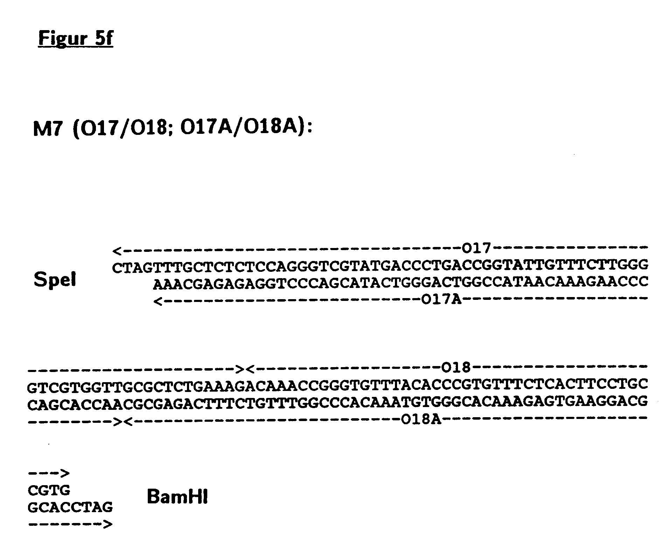

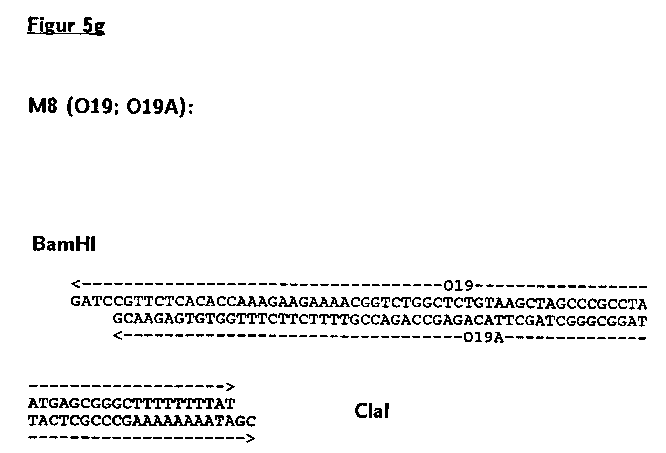

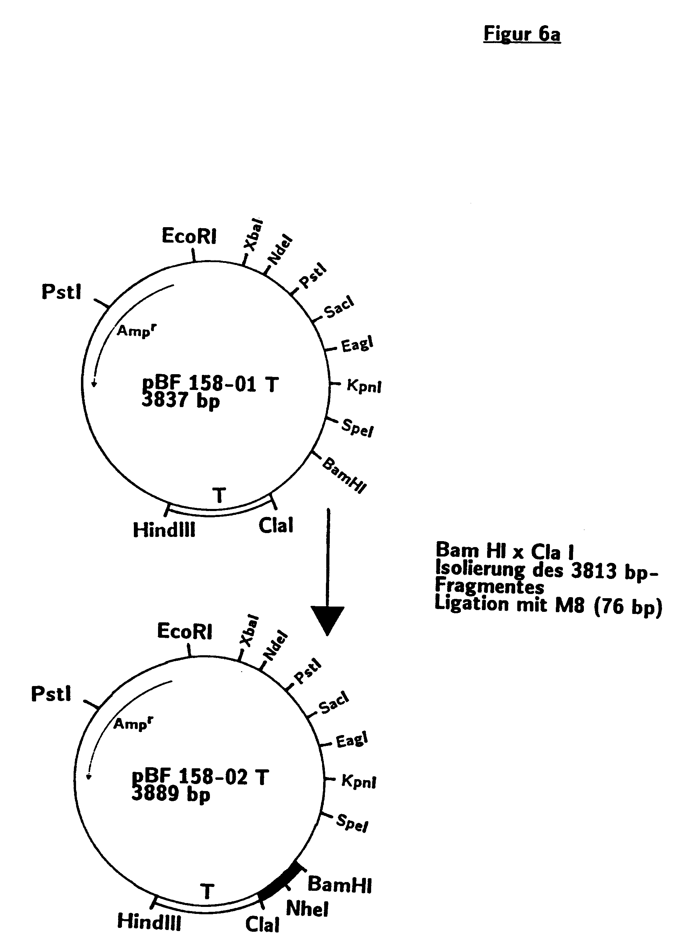

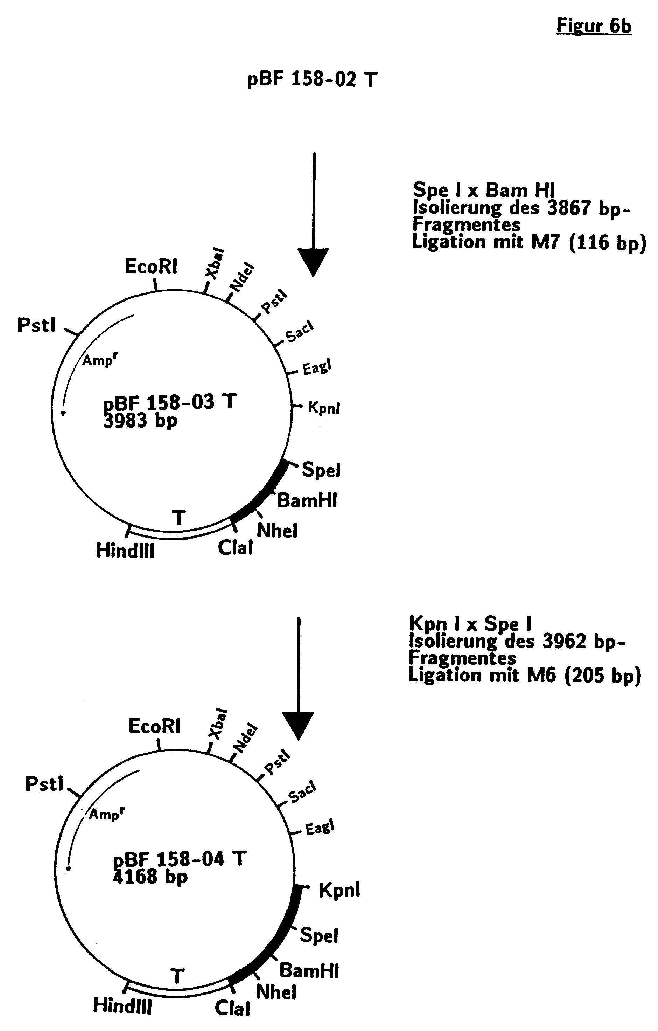

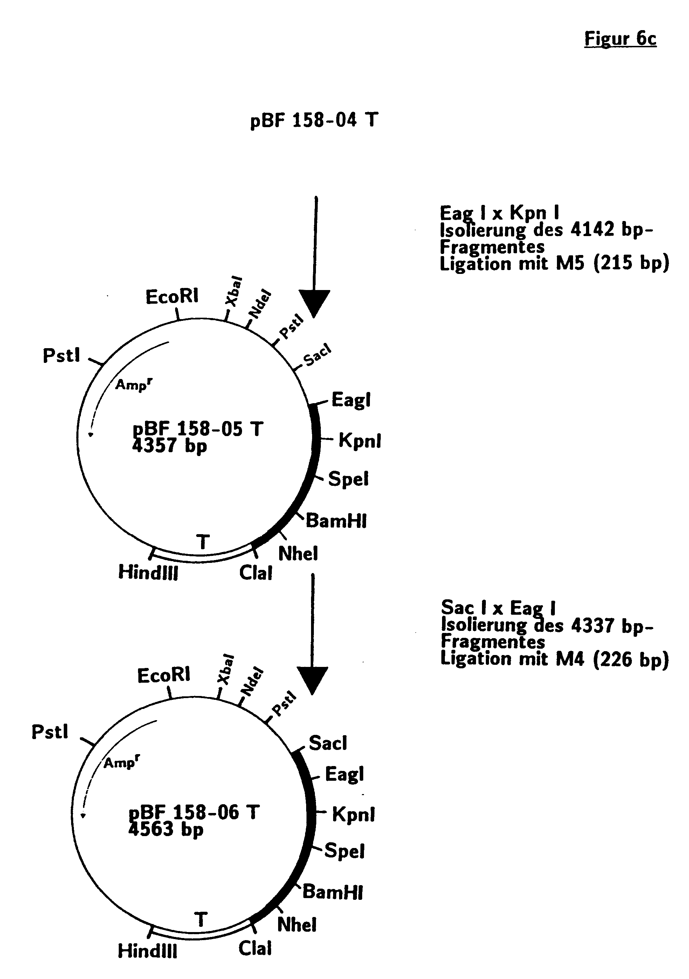

Konstruktion des Expressionsplasmids pBF 160 für das Vorstufenprotein des rscu-PA mit synthetischem scu-PA-Gen unter Kontrolle des Trp-Promotors.
a) Konstruktion des Plasmids pBF 158
   i) Aus dem Plasmid pBR 322 (Pharmacia, Nr. 27-4902, 4363 bp) wird die nic/bom Region, die sich zwischen den Basen 2207 und 2263 befindet (1), wie folgt entfernt (vgl. auch Figur 1):
      pBR 322 wird bei Base 2298 mit Nde I geschnitten und damit linearisiert. Die überstehenden Enden werden über eine "fill in" Reaktion aufgefüllt, und somit werden blunt-Enden erhalten (2). Danach wird bei Base 2068 mit Pvu II geschnitten und die beiden blunt Enden des verbleibenden Teils von pBR 322 nach üblicher Technik mit T4-Ligase wieder ligiert. Anschließend wird der Ligationsansatz in kompetente E.coli K12 JM 103 Zellen (ATCC 39403) transformiert (3). Die transformierten Zellen werden auf Medium unter Zusatz von 150 »g Ampicillin/ml kultiviert. Aus den erhaltenen Klonen werden diejenigen ausgewählt, die das Plasmid pBF 157 enthalten, das sich vom Ausgangsplasmid pBR 322 durch um 228 Nukleotide kleinere a) Pst I x BspM II -, b) Pst I x Bal I-, c) Pst I x Ava I-Fragmente unterscheidet. Die beiden singulären Schnittstellen PvuII und Nde I des pBR 322 sind in Plasmid pBF 157 nicht mehr vorhanden.
   ii) Aus pBF 157 wird ein großer Teil des Tetracyclinresistenzgens durch Schneiden mit Eco RV x Nru I und anschließende Ligation der entstehenden blunt Enden entfernt. Nach Transformation in E.coli K12 JM 103 und Kultivierung auf Medium mit 150 »g Ampicillin/ml werden Klone erhalten, von denen diejenigen ausgewählt werden, die das Plasmid pBF 158 enthalten. Dieses ist 787 Nukleotide kleiner als pBF 157 und unterscheidet sich außerdem durch das Fehlen einer Bam HI-Schnittstelle. Transformation von Bakterienstämmen mit pBF 158 verleiht diesen keine Tetracyclinresistenz.
b) Konstruktion von pBF 158-01, Einbau einer synthetischen multicloning site
   Aus pBF 158 wird durch Schneiden mit Eco RI x Hind III ein 31 Nukleotide großes Fragment entfernt und in die Lücke eine synthetische Multi cloning site ligiert, deren Sequenz in Figur 2 dargestellt ist. Nach Transformation des Ligationsansatzes in E.coli K12 JM103 und Kultivierung auf Medium mit 150 »g Ampicillin/ml werden diejenigen Klone ausgewählt, die das Plasmid pBF 158-01 enthalten. Dieses unterscheidet sich von pBF 158 durch die zusätzlichen singulären Schnittstellen Xba I, Nde I, Sac I, Eag I, Kpn I, Spe I sowie eine zweite Pst I-Schnittstelle (Fig. 3).
c) Konstruktion von pBF 158-01 T, Einbau eines Transkriptionsterminators
   Aus pBF 158-01 wird das Cla I x Hind III Fragment der Multi cloning site entfernt und durch das Cla I x Hind III Fragment aus pRT 61 (5), auf welchem sich der tet A/orf L Terminator aus Tn 10 (6) befindet, ersetzt.
   Nach Transformation in den Stamm E.coli K12 JM103 und Kultivierung auf Medium mit 150 »g Ampicillin/ml werden diejenigen Klone ausgewählt, die das Plasmid pBF 158-01 T enthalten. Dieses unterscheidet sich von pBF 158-01 durch ein um 297 Nukleotide größeres Cla I x Hind III Fragment (Fig. 4).
d) Einbau der synthetischen Fragmente M 4 - M 8 für das scu-PA-Gen, Konstruktion der Plasmide pBF 158-02 T - pBF 158-06 T
   Alle synthetischen Fragmente sind in Figuren 5a - 5g beschrieben. Sie werden als ca. 200 Nukleotide lange Doppel-Strang-Fragmente in die betreffenden Vektoren eingesetzt. Dazu werden die Vektoren mit den den jeweilig genannten Schnittstellen entsprechenden Restriktionsenzymen geschnitten und durch Agarosegelelektrophorese, Elektroelution und Reinigung über DE 52 vom zu entfernenden Fragment getrennt. Danach erfolgt Ligation mit dem zugehörigen doppelsträngigen synthetischen Fragment mit Hilfe von T4-Ligase, Transformation in E.coli K12 JM 103 (Fig. 6a - 6c) und Selektion auf Ampicillin-haltigem Medium.
   i) Ligation des Fragmentes M 8 zwischen Bam HI und Cla I im Plasmid pBF 158-01 T.
      Es entsteht das Plasmid pBF 158-02 T.
      Oligonukleotid 019 codiert für die C-terminale Sequenz des scu-PA. Hinter dem Stopp-Codon TAA befindet sich eine Nhe I-Schnittstelle, gefolgt von zusätzlichen Transkriptionsterminations-Sequenzen des trpA Terminators (8) bis Cla I.
   ii) Ligation des Fragmentes M 7 zwischen Spe I und Bam HI im Plasmid pBF 158-02 T.
      Es entsteht das Plasmid pBF 158-03 T.
   iii) Ligation des Fragmentes M 6 zwischen Kpn I und Spe I im Plasmid pBF 158-03 T.
      Es entsteht pBF 158-04 T.
   iv) Ligation des Fragmentes M 5 zwischen Eag I und Kpn I im Plasmid pBF 158-04 T.
      Es entsteht das Plasmid pBF 158-05 T.
   v) Ligation des Fragmentes M 4 zwischen Sac I und Eag I im Plasmid pBF 158-05 T.
      Es entsteht pBF 158-06 T.

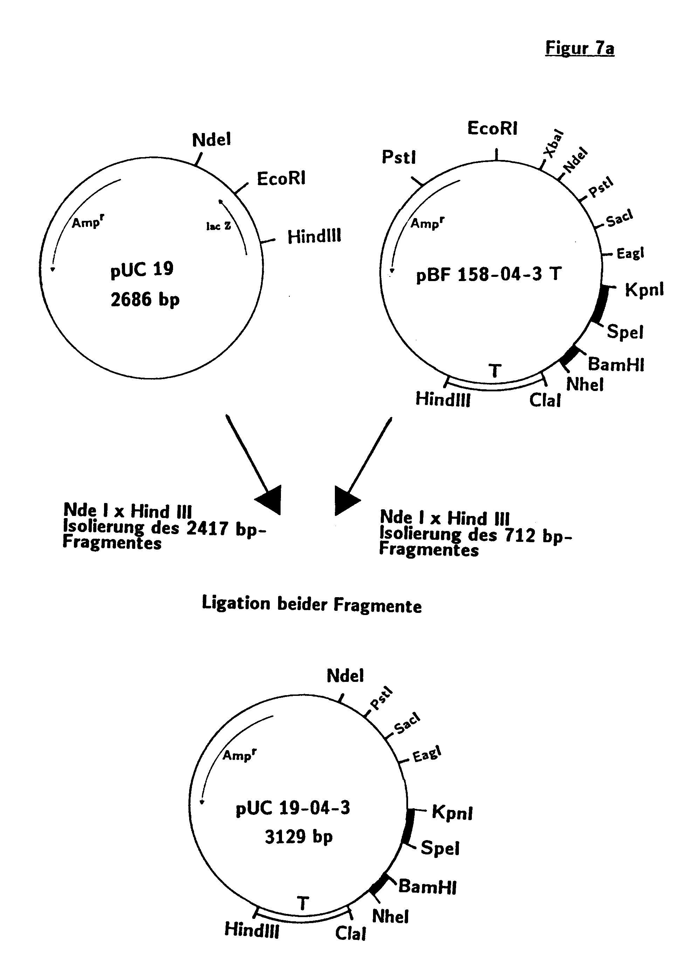

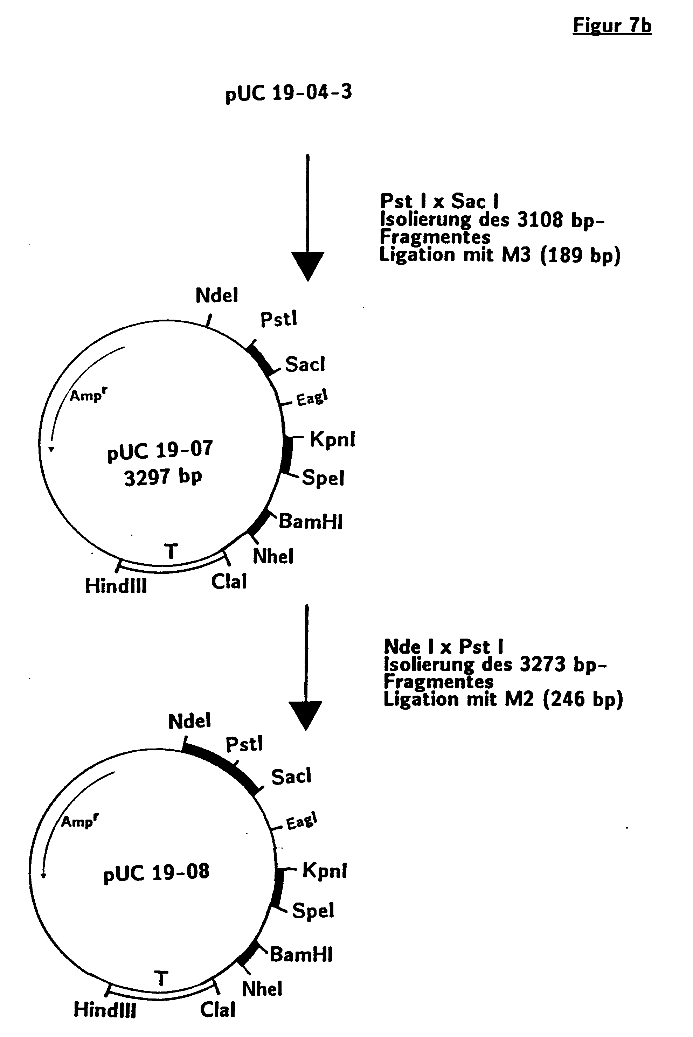

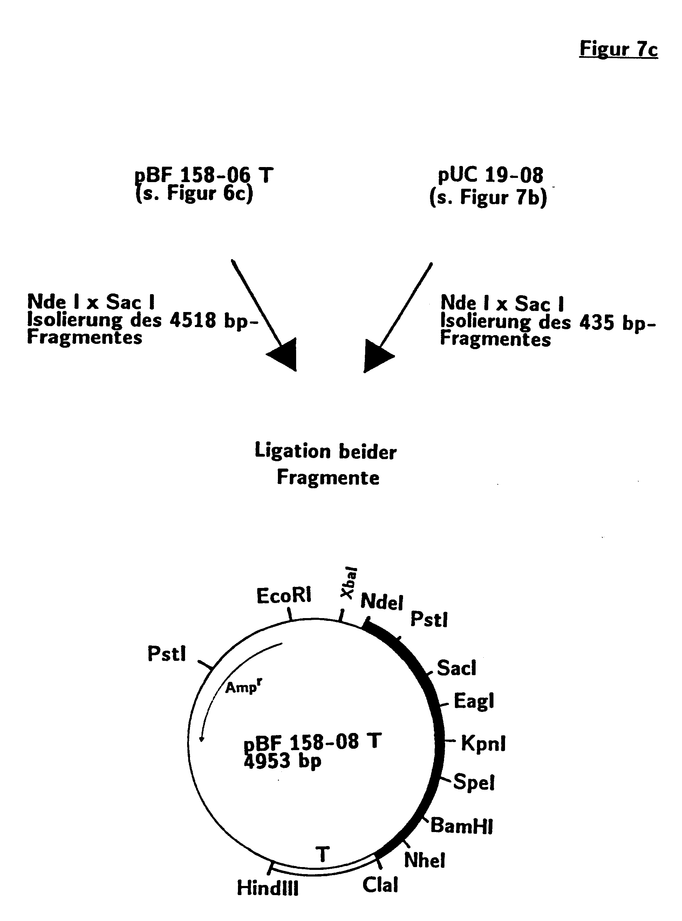

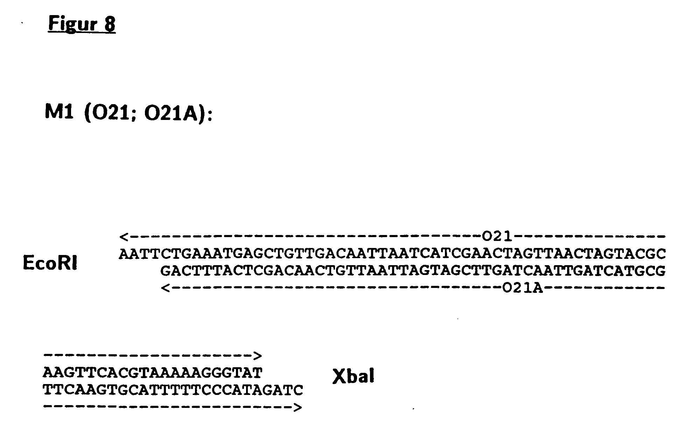

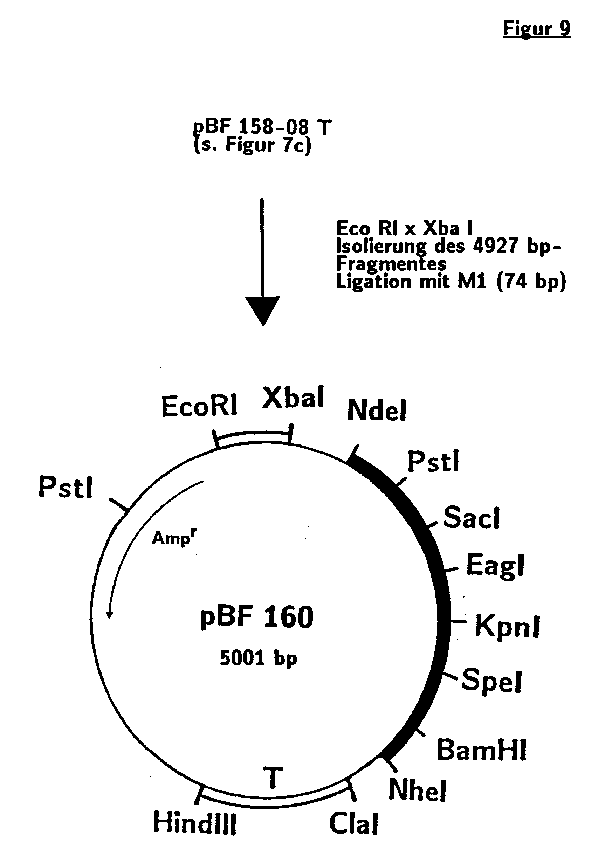

   Von den erhaltenen Klonen i - v werden jeweils diejenigen ausgewählt, die sich vom jeweiligen Vorläufer durch das Vorhandensein des eingebauten neuen Fragmentes in der überprüften richtigen Sequenz unterscheiden.
e) Einbau der synthetischen Fragmente M 2 und M 3 für das scu-PA-Gen Konstruktion von pBF 158-08 T
   Da für den Einbau der Fragmente M 2 und M 3 ein Pst I-Schnitt erforderlich ist und sich auf dem bis hierhin benutzten Plasmid pBF 158 noch eine Pst I Schnittstelle (im Ampicillinresistenzgen) befindet, die bei den folgenden Klonierungen stören würde, wird wie folgt vorgegangen (Figuren 7a bis 7c):
   i) Aus dem handelsüblichen (z. B. Pharmacia) Plasmid pUC 19 werden die Multi cloning site und zusätzlich 212 Nukleotide stromaufwärts als Nde I x Hind III Fragment entfernt. In die entstehende Lücke wird dann die synthetische Multi cloning site aus dem Plasmid pBF 158-04-3 T als Nde I x Hind III Fragment ligiert. Dieses Plasmid pBF 158-04-3 T wird aus dem Plasmid pBF 158-02 T (vgl. Beispiel 1di) durch Einbau des Oligonukleotidfragmentes M 6 erhalten und entspricht dem Plasmid pBF 158-04 T ohne das Fragment M 7. Nach Transformation in E.coli K 12 JM 103 Zellen und Kultivierung auf Medium mit 150 »g Ampicillin/ml werden diejenigen Klone ausgewählt, die das Plasmid pUC 19-04-3 enthalten. Dieses unterscheidet sich von pUC 19 durch das Vorhandensein eines 712 Nukleotide langen Nde I x Hind III Fragmentes.
   ii) Aus pUC 19-04-3 wird das Pst I x Sac I Fragment entfernt, der linearisierte Vektor wird isoliert und mit dem Oligonukleotidfragment M 3 ligiert. Nach Transformation in E.coli K12 JM 103 und Kultivierung auf Medium mit 150 »g Ampicillin/ml werden diejenigen Klone ausgewählt, die das Plasmid pUC 19-07 enthalten. Dieses unterscheidet sich von pUC 19-04-3 durch ein 189 Nukleotide langes Pst I x Sac I Fragment, das das Fragment M 3 mit der richtigen Sequenz enthält.
   iii) Aus dem vorstehend beschriebenen Plasmid pUC 19-07 wird das Nde I x Pst I Fragment entfernt und in die Lücke das Fragment M 2 ligiert. Nach Transformation in E.coli K12 JM 103 und Kultivierung auf Medium mit 150 »g Ampicillin/ml werden diejenigen Klone ausgewählt, die das Plasmid pUC 19-08 enthalten. Dieses unterscheidet sich von pUC 19-07 durch das Vorhandensein des 246 bp langen Nde I x Pst I Fragmentes M 2 mit der richtigen überprüften Sequenz.
   iv) Aus dem Plasmid pUC 19-08 werden die Fragmente M 2 und M 3 als Nde I x Sac I Fragment entfernt und in den nach Schnitten mit Nde I x Sac I erhaltenen größeren Teil von pBF 158-06 T ligiert. Nach Transformation in E.coli K12 JM 103 und Kultivierung auf Medium mit 150 »g Ampicillin/ml werden diejenigen Klone ausgewählt, die das Plasmid pBF 158-08 T enthalten. Dieses unterscheidet sich von pBF 158-06 T durch das Vorhandensein eines 435 Nukleotide langen Nde I x Sac I Fragmentes.
f) Konstruktion des Plasmids pBF 160, Einbau des synthetischen Trp-Promotors
   Die in (9) beschriebene Sequenz des Trp-Promotors wird bis zur Xba I-Schnittstelle übernommen und am 5′-Ende durch die Sequenz 5′-AATTCTGAAAT-3′ verlängert. Dadurch wird eine Eco RI-Schnittstelle konstruiert. (Fig. 8, Fragment M 1). Die Einzelstränge 021 und 021 A werden annealt und in das Eco RI x Xba I geschnittene Plasmid pBF 158-08 T ligiert (Fig. 9). Nach Transformation in E.coli K12 JM 103 und Kultivierung auf Medium mit 150 »g Ampicillin/ml werden diejenigen Klone ausgewählt, die pBF 160 enthalten. Das Plasmid pBF 160 unterscheidet sich von allen oben beschriebenen Vorläufern dadurch, daß damit transformierte E.coli Bakterienstämme auf Zugabe von Indolacrylsäure das Vorstufenprotein des rscu-PA exprimieren.
g) Expressionstest
   i) Fermentation
      Verschiedene, mit dem Plasmid pBF 160 transformierte E.coli-Stämme, z. B. E.coli GRT-1, E.coli K12 JM103 sowie E.coli K12 ATCC 31446, werden unter jeweils gleichen Bedingungen in einem aus 38 mM Ammoniumsulfat, 56 mM Phosphatpuffer pH 7,0, 1 mM Magnesiumsulfat, 1 % Hefeextrakt, 1 % Glucose bestehenden Medium, das 150 mg Ampicillin pro Liter enthält, fermentiert und mit 62 mg Indolacrylsäure/l wird die Expression des Vorstufenproteins des rscu-PA induziert. Zum Vergleich werden die vorstehend genannten Stämme mit einem vorbeschriebenen Plasmid, welches das Gen für menschliche Prourokinase aus einer cDNA-Bank, gewonnen aus Detroit 562 Zellen-mRNA (10) enthält, und das die Bezeichnung pUK 54 trp 207-1 trägt, transformiert und dann unter den gleichen Bedingungen fermentiert und der Induktion unterworfen.
      Vor der Induktion und jede Stunde nach der Induktion für insgesamt 6 Stunden werden Zellen entsprechend 1 ml einer Zellsuspension mit der optischen Dichte (OD) von 1 bei 578 nm abzentrifugiert und für die Testung der Expressionsrate eingesetzt.
   ii) Rückfaltung des Vorstufenproteins zum rscu-PA, dessen Spaltung zu rtcu-PA und Aktivitätsmessung.
      Die abzentrifugierten Zellen werden, wie hierin weiter oben beschrieben, mit Lysozym aufgeschlossen und dann wird das Homogenat der lysierten Zellen zur Aktivitätsbestimmung wie oben beschrieben eingesetzt.
      Die so nach Aktivitätsmessung des aus dem rscu-PA (erhalten aus dem Vorstufenprotein) gebildeten rtcu-PA ermittelten Expressionsraten sind in Figur 10 dargestellt. Daraus zeigt sich, daß die Stämme von E.coli, die mit dem Plasmid pBF 160 transformiert wurden, eine 10 - 15mal höherere Expressionsausbeute lieferten als die mit dem literaturbekannten Plasmid pUK 54 trp 207-1 (10) transformierten gleichen E.coli-Stämme. Darüber hinaus ergibt sich, daß die Expressionsausbeute in allen Stämmen in Abhängigkeit von dem zur Transformation benutzten Plasmid bei etwa gleichen Werten liegt, d. h. daß insbesondere die mit dem Plasmid pBF 160 transformierten Stämme (unabhängig vom einzelnen E.coli-Stamm) stets eine um ein Vielfaches höhere Expressionsrate als die mit dem bekannten Plasmid pUK 54 trp 207-1 transformierten identischen Stämme erbrachten.

### Beispiel 2

a) Konstruktion des Expressionsplasmids pBF-161 für das Vorstufenprotein des rscu-PA mit synthetischem scu-PA-Gen unter Kontrolle des Trp-Promotors.
Das Plasmid pBR 322 wird mit Eco RI und Hind III geschnitten. Das entstehende 31 Nukleotide lange Fragment wird durch eine präparative Agarosegelelektrophorese abgetrennt. Der verbleibende Anteil von pBR 322 wird durch Elektroelution aus dem Gel eluiert und durch Chromatographie über DE 52 gereinigt.
Das Plasmid pBF 160 (Beispiel 1f) wird ebenfalls mit Eco RI und Hind III geschnitten und das 1684 Nukleotide lange Eco RI x Hind III Fragment, das das synthetische scu-PA-Gen mit allen Regulationseinheiten (Trp-Promotor) enthält, durch Agarosegelelektrophorese abgetrennt und wie oben beschrieben eluiert und gereinigt.
Die so erhaltenen Fragmente werden auf übliche Weise mit T4-Ligase ligiert und dann in E.coli K12 JM 103 transformiert. Nach Kultivierung auf Medium mit 150 »g Ampicillin/ml werden diejenigen Klone ausgewählt, die ein 1684 Nukleotide langes Eco RI x Hind III Fragment enthalten und die nach Zugabe von Indolacrylsäure rscu-PA-Vorstufenprotein produzieren. Diese Klone enthalten Plasmid pBF 161.
b) Expressionstest
E.coli GRT-1, E.coli K12 JM 103 und E.coli K 12 ATCC 31 446 werden mit pBF 161 transformiert und dann wird wie in Beispiel 1g) beschrieben fermentiert und das Expressionsergebnis getestet. Die Ausbeuten an rscu-PA-Vorstufenprotein bestimmt als rtcu-PA-Aktivität 1 - 6 Stunden nach Induktion sind vergleichbar mit den in Fig. 10 für die Verwendung von Plasmid pBF 160 zur Transformation dargestellten Ausbeutewerten.
Das analog Beispiel 1gi) durch Zentrifugation gewonnene Zell-Pellet kann auch durch Kochen in 0,25M Tris HCl-Puffer, pH 8,0 mit 4 % SDS, 1 % Mercaptoethanol und 20 % Glycerin aufgeschlossen werden. Die so gelösten Proteine werden durch SDS-PAGE aufgetrennt und durch Coomassieblue-Färbung (12) sichtbar gemacht. Die gefärbten Gele werden densitometrisch ausgewertet und die Flächen unter den Peaks integriert. Der Anteil des nach Induktion mit Indolacrylsäure gebildeten rscu-PA-Vorstufenproteins wird durch Subtraktion der Fläche der als Vorstufenprotein identifizierten Bande vor Induktion von der nach Induktion erhaltenen ermittelt. In Fig. 11 ist ein Densitogramm für aus E.coli K 12 JM 103 Zellen erhaltene Proteine dargestellt. Im vorliegenden Beispiel beträgt der Anteil von rscu-PA-Vorstufenprotein nach Induktion 17,9 Gewichtsprozent des bakteriellen Gesamtproteins. Weitere Beispiele sind in Tabelle 1 angegeben.

**Tabelle 1**

| Anteil des rscu-PA-Vorstufenproteins am Gesamtprotein (in Prozent) | | |
|---|---|---|
| E.coli-Stamm | transformiert mit | |
| | pBF 161 | pUK54 trp 207-1 (10) |
| K 12 ATCC 31446 | 14 | 1,5 |
| K 12 JM 103 | 17,9 | 1,9 |
| GRT-1 | 17,8 | 1,7 |

### Beispiel 3

Konstruktion eines Expressionsplasmids für das Vorstufenprotein des rscu-PA mit synthetischem scu-PA-Gen unter Kontrolle des Trp-Promotors in pBR 322 mit Deletion im Tetracyclinresistenzgen.
a) Konstruktion des Plasmids pBR 322 del
   Das Plasmid pBR 322 wird mit Eco RV und Nru I geschnitten. Das entstehende 787 Nukleotide große Fragment wird durch Agarosegelelektrophorese entfernt, der Restanteil des pBR 322 durch Elektroelution aus dem Gel eluiert und über DE 52 gereinigt. Die blunt-Enden des pBR 322 Eco RV x Nru I-Restanteils werden auf übliche Weise mit T4-Ligase ligiert. Nach Transformation in E.coli K12 JM 103 und Kultivierung auf Medium mit 150 »g Ampicillin/ml werden diejenigen Klone ausgewählt, von denen ein aliquoter Teil nach Übertragung auf ein Medium mit 25 »g Tetracyclin/ml auf diesem nicht wächst, d. h. keine Tetracyclinresistenz besitzt. Die Klone enthalten das Plasmid pBR 322 del, das sich von pBR 322 dadurch unterscheidet, daß es 787 Nukleotide kleiner ist und sich von Eco RV und Nru I nicht mehr schneiden läßt.
b) Konstruktion des Plasmids pBF 162
   pBR 322 del wird mit Eco RI und Hind III geschnitten. Das entstehende 31 Nukleotide lange Fragment wird durch eine präparative Agarosegelelektrophorese abgetrennt, der Restanteil von pBR 322 del wird durch Elektroelution aus dem Gel eluiert und über DE 52 gereinigt.
   Aus pBF 160 wird das 1684 Nukleotide lange Eco RI x Hind III Fragment, das das synthetische scu-PA-Gen mit allen Regulationseinheiten (Trp-Promotor) enthält, auf die gleiche Art gewonnen.
   Beide Fragmente werden auf übliche Art mit T4-Ligase ligiert und dann in E.coli K12 JM 103 Zellen transformiert. Nach Kultivierung auf Medium mit 150 »g Ampicillin/ml werden die Klone ausgewählt, die ein 1684 bp langes Eco RI x Hind III Fragment enthalten und die nach Zugabe von 62 »g Indolacrylsäure/ml rscu-PA-Vorstufenprotein produzieren. Diese Klone enthalten Plasmid pBF 162.
c) Expressionstest
   E.coli GRT-1 wird mit pBF 162 transformiert, und dann wird wie in Beispiel 1g) beschrieben fermentiert und das Expressionsergebnis getestet. Die Ausbeute an rscu-PA-Vorstufenprotein bestimmt als rtcu-PA-Aktivität 6 Stunden nach Induktion beträgt 1300 PU/ml einer Zellsuspension mit der optischen Dichte 1 und ist vergleichbar mit den in Fig. 10 für die Expression nach Transformation von E.coli GRT-1 mit dem Plasmid pBF 160 dargestellten Ausbeutewerten.

### Beispiel 4

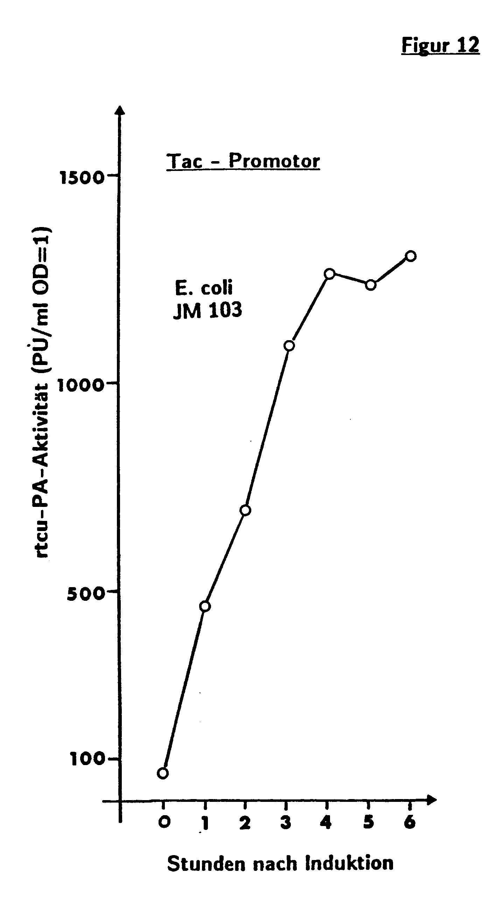

a) Konstruktion des Expressionsplasmids pBF 171 für das Vorstufenprotein des rscu-PA mit synthetischem scu-PA-Gen unter Kontrolle des Tac Promotors.
   Das Plasmid pBF 161 wird mit Eco RI und Xba I geschnitten. Das entstehende 74 Nukleotide lange Fragment wird durch präparative Agarosegelelektrophorese abgetrennt, der Restanteil des Plasmids durch Elektroelution eluiert und dann über DE 52 gereinigt.
   Aus dem Plasmid ptac SDT (DSM 5018) wird das Eco RI x Xba I Fragment, das den Tac-Promotor enthält, isoliert. Hierzu wird ptac SDT mit Eco RI x Xba I geschnitten und das Fragment durch präparative PAGE abgetrennt. Das Fragment wird durch Erhitzen auf 65° C in Ammoniumacetat/SDS-Puffer, pH 8,0, aus dem mechanisch zerkleinerten Polyacrylamid eluiert und durch mehrmalige Extraktion mit Phenol, das mit 1 M Tris-Puffer, pH 8, gesättigt ist, gereinigt.
   Beide so gewonnenen Fragmente werden auf übliche Weise mit T4-Ligase ligiert und dann in E.coli K 12 JM 103 transformiert. Nach Kultivieren auf Medium mit 150 »g Ampicillin/ml werden die Klone ausgewählt, die nach Zugabe von IPTG rscu-PA-Vorstufenprotein produzieren. Diese Klone enthalten das Plasmid pBF 171.
b) Expressionstest
   E.coli K 12 JM 103 wird mit pBF 171 transformiert, und dann wird wie in Beispiel 1g) beschrieben fermentiert und das Expressionsergebnis getestet. Die Induktion erfolgt jedoch mit IPTG (Endkonzentration 0,5 mM).
   Die Ausbeuten an rscu-PA-Vorstufenprotein, bestimmt als rtcu-PA-Aktivität 1 - 6 Stunden nach Induktion sind in Fig. 12 dargestellt. Sie sind mit den in Fig. 10 für die Verwendung von Plasmid pBF 160 im gleichen E.coli-Stamm dargestellten Ausbeutewerten vergleichbar.

### Beispiel 5

a) Konstruktion des Expressionsplasmids pBF 172 für das rscu-PA-Vorstufenprotein mit synthetischem scu-PA-Gen unter Kontrolle des Tac Promotors in pBR 322 mit Deletion im Tetracyclinresistenzgen.
   Das Plasmid pBR 322 del (siehe Beispiel 3a) wird mit Eco RI x Hind III geschnitten. Das entstehende 31 Nukleotide lange Fragment wird durch eine präparative Agarosegelelektrophorese abgetrennt, der pBR 322 del-Restanteil durch Elektroelution aus dem Gel eluiert und dann über DE 52 gereinigt.
   Aus pBF 171 (Beispiel 4a) wird das Eco RI x Hind III Fragment, das das synthetische scu-PA-Gen mit allen Regulationseinheiten (Tac-Promotor) enthält, auf die gleiche Art gewonnen.
   Beide so erhaltenen Fragmente werden auf übliche Weise mit T4 Ligase ligiert und dann in E.coli K 12 JM 103 Zellen transformiert. Nach Kultivierung auf Medium mit 150 »g Ampicillin/ml werden die Klone ausgewählt, die in Gegenwart von 0,5 mM IPTG rscu-PA-Vorstufenprotein produzieren. Diese Klone enthalten das Plasmid pBF 172.
b) Expressionstest
   E.coli K12 JM 103 wird mit pBF 172 transformiert, und dann wird wie in Beispiel 1g) beschrieben fermentiert und das Expressionsergebnis getestet. Die Induktion erfolgt jedoch mit IPTG (Endkonzentration 0,5 mM). Die Ausbeuten an rscu-PA-Vorstufenprotein bestimmt als rtcu-PA-Aktivität 1 - 6 Stunden nach Induktion betragen etwa 1100 PU/ml Zellsuspension der optischen Dichte 1 und sind mit den in Fig. 10 für die Verwendung von Plasmid pBF 160 im gleichen E.coli-Stamm dargestellten Ausbeutewerten vergleichbar.

### Beispiel 6

Veränderung des Abstandes zwischen Shine Dalgarno Sequenz und Startcodon in einem Expressionsplasmid für rscu-PA-Vorstufenprotein.

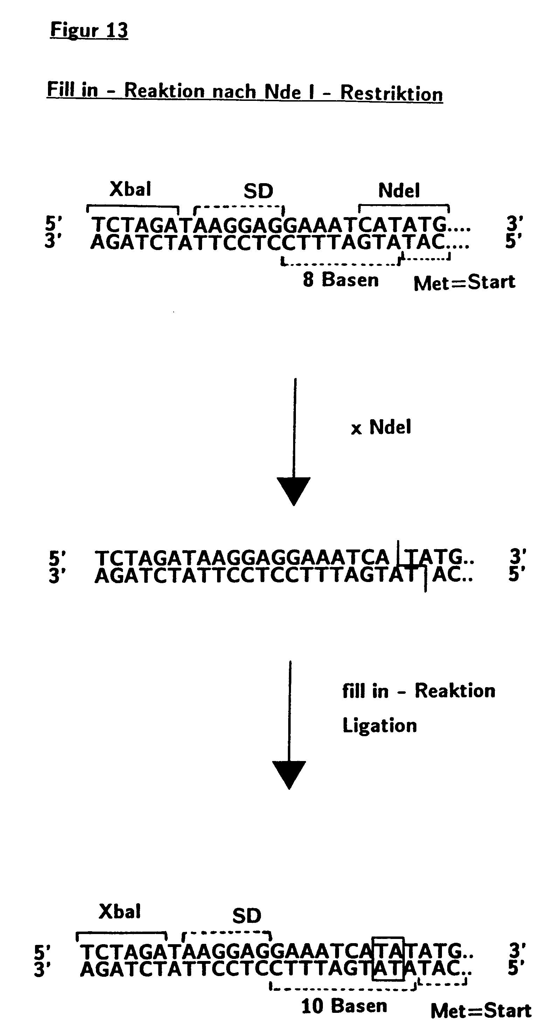

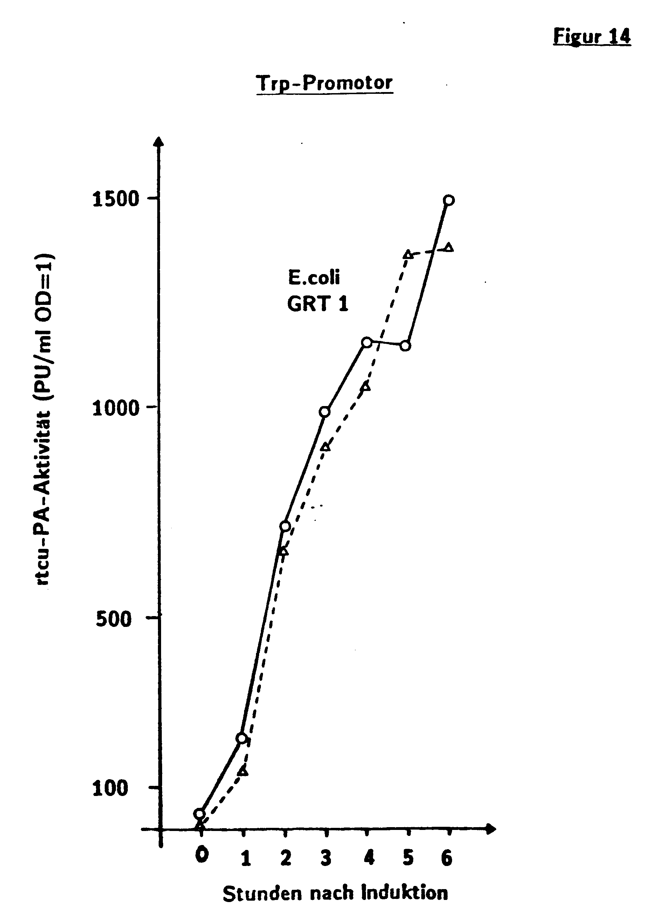

Das Startcodon ATG in allen in Beispielen 1 - 5 beschriebenen Konstruktionen ist Bestandteil einer Nde I-Schnittstelle -CATATG-. Nde I schneidet hinter dem ersten A der Hexanukleotidsequenz und liefert 5'-Enden, die um zwei Basen überstehen. Füllt man das 3'-Ende auf, d. h. konstruiert man so blunt-Enden, und ligiert danach wieder, ist die betreffende Sequenz um 2 Basenpaare verlängert. Gleichzeitig ist die Nde I-Stelle eliminiert. Wie aus Fig. 13 ersichtlich, wird in dem dort dargestellten Beispiel der Abstand von der S.D.-Sequenz bis zum Startcodon von 8 auf 10 Basenpaare verlängert. Im folgenden wird die experimentelle Durchführung in einem Beispiel erläutert:
a) Konstruktion des Plasmids pBF 163
   Das Plasmid pBF 160 wird mit Nde I geschnitten, und dann werden die überstehenden Enden mit dem Klenow-Fragment der DNA-Polymerase I aufgefüllt (2). Danach wird die DNA wie üblich mit T4-Ligase ligiert und dann in E.coli K 12 JM 103 transformiert. Nach Kultivierung auf ampicillinhaltigem Medium werden die Klone ausgewählt, die das Plasmid pBF 163 enthalten. Dieses unterscheidet sich von pBF 160 durch das Fehlen einer Nde I-Stelle und durch die zusätzlichen Basen -TA- im Bereich der früheren Nde I-Stelle (vgl. Fig. 13).
b) Expressionstest
   E.coli GRT-1 wird mit dem Plasmid pBF 163 transformiert, und dann wird wie in Beispiel 1g) beschrieben fermentiert und das Expressionsergebnis getestet. Die Ausbeuten an rscu-PA-Vorstufenprotein, bestimmt nach Rückfalten und Aktivierung als rtcu-PA-Aktivität pro ml Zellsuspension mit der optischen Dichte 1 ein bis sechs Stunden nach Induktion mit 62 mg Indolacrylsäure/l sind in Fig. 14 aufgeführt. Sie sind mit den in Fig. 10 für die Verwendung von Plasmid pBF 160 im gleichen Stamm von E.coli dargestellten Ausbeutewerten vergleichbar.

In der folgenden Tabelle 2 sind die oben genannten hinterlegten Mikroorganismen zusammengefaßt:

| Mikroorganismus | Hinterlegungs- | |
|---|---|---|
| | stelle | nummer |
| pBR 322 | ATCC | 31 344 |
| ptacSDT (in E.coli K12 JM 103) | DSM | 5 018 |
| E.coli K12 JM 101 | ATCC | 33 876 |
| E.coli K12 JM 103 | ATCC | 39 403 |
| E.coli K12 JM 105 | DSM | 4 162 |
| E.coli K12 DH 1 | ATCC | 33 849 |
| E.coli K12 | ATCC | 31 446 |

### Referenzen

( 1) Winnacker, E.L.: "Gene und Klone", VCH Verlagsgesellschaft, Weinheim, 1985, S. 298
( 2) Maniatis, T., Fritsch, E.F., Sambrook, J.: "Molecular Cloning", A Laboratory Manual, Cold Spring Harbor Laboratory, 1982
( 3) Hanahan, I.: "DNA cloning", Vol. I, ed. D.M. Glover, IRL Press, Oxford 1985, S. 109 - 135
( 4) Wilhelm, M., Hollenberg, C.P.: Nucl. Acids Res. 13, 5717 - 5722 (1985)
( 5) Jorgensen, R.A., Reznikoff, W.S.: J. Bacteriol. 138, 705 - 714 (1979)
( 6) Schollmeier, K., Gärtner, D., Hillen, W.: Nucl. Acids Res. 13, 4227 - 4237 (1985)
( 7) Adams, S.P., Kavka, K.S., Wykes, E.I., Holder, S.B., Gallupi, G.R.: J. Am. Chem. Soc. 105, 661 - 663 (1983)
( 8) Christie, G.E., Farnham, P.J., Platt, T.: Proc. Natl. Acad. Sci, USA 78, 4180 - 4184 (1981)
( 9) De Boer, H.A., Comstock, L.J., Vasser, M.: Proc. Natl. Acad. Sci. USA 80, 21 - 25 (1983)
(10) Holmes, W.E., Pennica, D., Blaber, M., Rey M.W., Günzler, W.A., Steffens, G.J., Heynecker, H.L.: Biotechnol. 3, 923 - 929 (1985)
(11) Winkler, M.E., Blaber, M.: Biochem. 25, 4041 - 4045 (1986)
(12) Blakesley, R.W., Boezi, J.A.: Anal. Biochem. 82 580 - 582 (1977)

## Patentansprüche

1. Plasmide zur Verwendung bei der Gewinnung eines Plasminogenaktivators, dadurch gekennzeichnet, daß ihr Operon
einen Trp- oder Tac-Promotor als regulierbaren Promotor,
eine als Ribosomenbindestelle wirksame Shine-Dalgarno-Sequenz,
im Abstand von 6 bis 12 Nukleotiden zur Shine-Dalgarno-Sequenz ein Startcodon,
ein synthetisches Strukturgen für den 411 Aminosäurereste enthaltenden einkettigen Urin-Plasminogenaktivator "scu-PA", in dem als Codons für Arginin das Triplett CGT, für Leucin das Triplett CTG, für Valin das Triplett GTT, für Prolin das Triplett CCG und/oder für Glycin das Triplett GGT Verwendung finden, und
stromabwärts vom Strukturgen einen oder zwei Terminatoren, ausgewählt aus der Gruppe trp A Terminator und/oder tet A/orf L-Terminator aus Tn 10,
aufweist und daß sie zur rscu-PA-Vorstufenprotein-Expression in Enterobakteriaceen, vorzugsweise in Stämmen von Escherichia coli, geeignet sind.

2. Plasmide gemäß Anspruch 1, dadurch gekennzeichnet, daß der Abstand zwischen der Shine-Dalgarno-Sequenz und dem Startcodon 8 bis 10 Nukleotide beträgt.

3. Plasmide gemäß Anspruch 1, dadurch gekennzeichnet, daß der regulierbare Promotor ein synthetischer Trp-Promotor mit der Nukleotidsequenz gemäß Figur 8 ist.

4. Plasmide gemäß Anspruch 1, dadurch gekennzeichnet, daß der regulierbare Promotor der Tac-Promotor aus dem Plasmid ptac SDT (DSM 5018) ist.

5. Plasmide gemäß den Ansprüchen 1 bis 4, dadurch gekennzeichnet, daß das Strukturgen die Nukleotidsequenz gemäß Figur 15 hat.

6. Plasmide gemäß den Ansprüchen 1 bis 5, dadurch gekennzeichnet, daß sie das Operon in dem Plasmid pBR 322 tragen, aus dem die nic/bom-Region entfernt ist.

7. Plasmide gemäß Ansprüchen 1 bis 6, dadurch gekennzeichnet, daß sie das Operon in dem Plasmid pBR 322 tragen, aus dem das Tetracyclinresistenzgen ganz oder teilweise soweit entfernt ist, daß das Plasmid damit transformierten Bakterienstämmen keine Tetracyclinresistenz zu verleihen vermag.

8. Plasmide gemäß Ansprüchen 1 bis 7, dadurch gekennzeichnet, daß sie das Operon in dem Plasmid pBR 322 tragen, aus dem die nic/bom-Region und zumindest ein solcher Teil des Tetracyclinresistenzgens entfernt ist, daß das Plasmid damit transformierten Bakterienstämmen keine Tetracyclinresistenz zu verleihen vermag.

9. Als Plasmide gemäß Ansprüchen 1 und 3 die Plasmide pBF 160 mit der Restriktionskarte gemäß Figur 9, pBF 161, hergestellt wie in Beispiel 2 beschrieben, pBF 162, hergestellt wie in Beispiel 3 beschrieben und pBF 163, hergestellt wie in Beispiel 6 beschrieben.

10. Als Plasmide gemäß Ansprüchen 1 und 4 die Plasmide pBF 171, hergestellt wie in Beispiel 4 beschrieben und pBF 172, hergestellt wie in Beispiel 5 beschrieben.

11. Plasmide gemäß Ansprüchen 1 bis 10 mit dem weiteren Merkmal, daß sie in Stämmen von E.coli, vorzugsweise der Untergruppe E.coli K12, die Bildung des rscu-PA-Vorstufenproteins mit einer Expressionsrate von wenigstens 10 Gewichtsprozent, vorzugsweise von wenigstens 14 Gewichtsprozent des gebildeten Gesamtproteins bewirken.

12. Plasmide gemäß Ansprüchen 1 bis 11 mit dem weiteren Merkmal, daß sie in Stämmen von E.coli, vorzugsweise der Untergruppe E.coli K12, die Bildung des rscu-PA-Vorstufenproteins mit einer Expressionsrate von 10 bis 25 Gewichtsprozent, insbesondere von 14 bis 20 Gewichtsprozent des gebildeten Gesamtproteins bewirken.

13. Verfahren zur Herstellung von Plasmiden gemäß Anspruch 1, dadurch gekennzeichnet, daß man in das Plasmid pBR 322, aus dem die nic/bom-Region und/oder zumindest ein Teil des Tetracyclinresistenzgens entfernt wurden, zwischen die Schnittstellen Eco RI und Hind III eine "Multi cloning site" mit der in Figur 2 angegebenen Nukleotidsequenz, die zwischen den Schnittstellen Xba I und Nde I die Shine-Dalgarno-Sequenz und in einem Abstand von 6 bis 12, vorzugsweise 8 bis 10 Nukleotiden das Startcodon ATG enthält, einsetzt und mit deren Hilfe in an sich bekannter Weise einen oder zwei Transkriptionsterminatoren, die synthetischen Teilsequenzen des scu-PA-Strukturgens, vorzugsweise beginnend vom 3'C-Terminus des Strukturgens aus, sowie einen regulierbaren Promotor einbaut.

14. Verfahren zur Herstellung von Plasmiden gemäß Anspruch 1, dadurch gekennzeichnet, daß man das Eco RI x Hind III Fragment aus einem gemäß Anspruch 13 erhaltenen Plasmid als Expressionskassette in ein anderes in Enterobacteriaceen, vorzugsweise in E.coli autonom vermehrungsfähiges Plasmid in an sich bekannter Weise einsetzt.

15. Verfahren zur Herstellung von Plasmiden gemäß Ansprüchen 1 und 2, dadurch gekennzeichnet, daß man ein gemäß Anspruch 13 oder 14 erhaltenes Plasmid mit Nde I schneidet, die resultierenden Schnittstellen auffüllt und anschließend die entstehenden blunt-Enden ligiert.

16. Verwendung eines Plasmids gemäß Ansprüchen 1 bis 12 bei der Gewinnung eines Plasminogenaktivators, dadurch gekennzeichnet, daß man mit dem Plasmid einen Enterobacteriaceen-Stamm, vorzugsweise einen Stamm von E.coli, in an sich bekannter Weise transformiert, die Expression des scu-PA-Strukturgens induziert, das gebildete rscu-PA-Vorstufenprotein vom Medium und den lysierten Bakterienzellen abtrennt, das Vorstufenprotein solubilisiert und dann durch Einwirkung eines Redox-Systems zum rscu-PA rückfaltet.

17. Verwendung gemäß Anspruch 16, dadurch gekennzeichnet, daß man mit dem Plasmid einen Stamm der Species E.coli, vorzugsweise einen E.coli-K12-Stamm transformiert.

## Claims

1. Plasmids for use in the obtainment of a plasminogen activator, characterised in that their operon comprises
a Trp or Tac promoter as the regulable promoter,
a Shine-Dalgarno sequence which acts as a ribosome binding location,
a start codon at a distance of 6 to 12 nucleotides from the Shine-Dalgarno sequence,
a synthetic structural gene for the 411 amino acid residues containing the single-chain urine plasminogen activator "scu-PA", in which the CGT triplet is used as the codon for arginine, the CTG triplet is used as the codon for leucine, the GTT triplet is used as the codon for valine, the CCG triplet is used as the codon for proline, and/or the GGT triplet is used as the codon for glycine, and
one or two terminators downstream of the structural gene, selected from the group comprising the trp A terminator and/or the tet A/orf L terminator from Tn 10,
and that they are suitable for rscu-PA preliminary stage protein expression in Enterobacteriaceae, preferably in strains of Escherichia coli.

2. Plasmids according to claim 1, characterised in that the distance between the Shine-Dalgarno sequence and the start codon is 8 to 10 nucleotides.

3. Plasmids according to claim 1, characterised in that the regulable promoter is a synthetic Trp promoter with a nucleotide sequence according to Figure 8.

4. Plasmids according to claim 1, characterised in that the regulable promoter is the Tac promoter from plasmid ptac SDT (DSM 5018).

5. Plasmids according to claims 1 to 4, characterised in that the structural gene has the nucleotide sequence according to Figure 15.

6. Plasmids according to claims 1 to 5, characterised in that they carry the operon in plasmid pBR 322, from which the nic/bom region is removed.

7. Plasmids according to claims 1 to 6, characterised in that they carry the operon in plasmid pBR 322, from which the tetracycline resistance gene is completely or partially removed to such an extent that the plasmid is thereby incapable of imparting any tetracycline resistance to transformed strains of bacteria.

8. Plasmids according to claims 1 to 7, characterised in that they carry the operon in plasmid pBR 322, from which the nic/bom region is removed, and at least part of the tetracycline resistance gene is completely or partially removed such that the plasmid is thereby incapable of imparting any tetracycline resistance to transformed strains of bacteria.

9. As plasmids according to claims 1 and 3, the plasmids pBF 160 with the restriction map according to Figure 9, pBF 161 prepared as described in Example 2, pBF 162 prepared as described in Example 3, and pBF 163 prepared as described in Example 6.

10. As plasmids according to claims 1 and 4, the plasmids pBF 171 prepared as described in Example 4, and pBF 172 prepared as described in Example 5.

11. Plasmids according to claims 1 to 10, with the additional feature that in strains of E.coli, preferably in E.coli subgroup K12, they effect the formation of the rscu-PA preliminary stage protein at an expression rate of at least 10 weight percent, preferably of at least 14 weight percent, of the total protein formed.

12. Plasmids according to claims 1 to 11, with the additional feature that in strains of E.coli, preferably in E.coli subgroup K12, they effect the formation of the rscu-PA preliminary stage protein at an expression rate of 10 to 25 weight percent, particularly from 14 to 20 weight percent, of the total protein formed.

13. A method of preparing plasmids according to claim 1, characterised in that a "multi cloning site" having the nucleotide sequence given in Figure 2, which contains the Shine-Dalgarno sequence between the Xba I and Nde I cleavage sites and contains the ATG start codon at a distance of 6 to 12, preferably 8 to 10, nucleotides, is inserted between the Eco RI and Hind III cleaving sites in plasmid pBR 322 from which the nic/bom region and/or at least part of the tetracycline resistance gene have been removed, and with the aid of the multi cloning site one or two transcription terminators, the synthetic partial sequences of the scu-PA structural gene, preferably starting from the 3'C terminus of the structural gene, and a regulable promoter are incorporated in a manner known in the art.

14. A method of preparing plasmids according to claim 1, characterised in that the Eco RI x Hind III fragment from a plasmid obtained according to claim 13 is inserted in a manner known in the art as an expression cassette in another plasmid capable of autonomous reproduction in Enterobacteriaceae, preferably in E.coli.

15. A method of preparing plasmids according to claims 1 and 2, characterised in that a plasmid obtained according to claim 13 or 14 is cleaved with Nde I, the resulting cleavage sites are filled in and the blunt ends arising are subsequently ligated.

16. Use of a plasmid according to claims 1 to 12 in the obtainment of a plasminogen activator, characterised in that an Enterobacteriaceae strain, preferably a strain of E.coli, is transformed with the plasmid in a manner known in the art, expression of the scu-PA structural gene is induced, the rscu-PA preliminary stage protein formed is separated from the medium and the lysed bacterial cells, the preliminary stage protein is solubilised and is then folded back to form rscu-PA by the action of a redox system.

17. A use according to claim 16, characterised in that a strain of the species E.coli, preferably an E.coli K12 strain, is transformed with the plasmid.

## Revendications

1. Plasmides destinés à être utilisés pour l'obtention d'un activateur de plasminogène, caractérisé en ce que son opéron présente
un Trp- ou Tac-promoteur comme promoteur réglable,
une séquence de Shine-Dalgarno efficace comme site de liaison ribosomique,
un codon d'initiation à une distance de 6 à 12 nucléotides de la séquence de Shine-Dalgarno,
un gène synthétique de structure pour l'activateur de plasminogène urinaire "scu-PA" monocaténaire contenant 411 restes d'aminoacides, dans lequel sont utilisés comme codons le triplet CGT pour l'arginine, le triplet CTG pour la leucine, le triplet GTT pour la valine, le triplet CCG pour la proline et/ou le triplet GGT pour la glycine, et
en aval du gène de structure, un ou deux terminateurs choisis dans le groupe comprenant le trp A terminateur et/ou le tet A/orf L-terminateur de Tn 10,
et en ce qu'ils conviennent pour l'expression de la protéine précurseur de rscu-PA dans des entérobactériacées, de préférence dans des souches d'Escherichia coli.

2. Plasmides suivant la revendication 1, caractérisés en ce que la distance entre la séquence de Shine-Dalgarno et le codon d'initiation s'élève à 8 - 10 nucléotides.

3. Plasmides suivant la revendication 1, caractérisés en ce que le promoteur réglable est un trp-promoteur synthétique ayant la séquence de nucléotides suivant la figure 8.

4. Plasmides suivant la revendication 1, caractérisés en ce que le promoteur réglable est le Tac-promoteur dérivé du plasmide ptac SDT (DSM 5018).

5. Plasmides suivant les revendications 1 à 4, caractérisés en ce que le gène de structure a la séquence de nucléotides suivant la figure 15.

6. Plasmides suivant les revendications 1 à 5, caractérisés en ce qu'ils portent l'opéron dans le plasmide pBR 322 duquel la région nic/bom est enlevée.

7. Plasmides suivant les revendications 1 à 6, caractérisés en ce qu'ils portent l'opéron dans le plasmide pBR 322 duquel le gène de résistance à la tétracycline est totalement enlevé ou enlevé en partie dans une mesure telle que le plasmide ne puisse pas conférer la résistance à la tétracycline à des souches bactériennes transformées avec lui.

8. Plasmides suivant les revendications 1 à 7, caractérisés en ce qu'ils portent l'opéron dans le plasmide pBR 322 duquel sont enlevés la région nic/bom et au moins une partie du gène de résistance à la tétracycline telle que le plasmide ne puisse pas conférer de résistance à la tétracycline à des souches bactériennes transformées avec lui.

9. A titre de plasmides suivant les revendications 1 et 3, les plasmides pBF 160 ayant la carte de restriction suivant la figure 9, pBF 161 préparé comme décrit dans l'Exemple 2, pBF 162 préparé comme décrit dans l'Exemple 3 et pBF 163 préparé comme décrit dans l'Exemple 6.

10. A titre de plasmides suivant les revendications 1 et 4, les plasmides pBF 171 préparé comme décrit dans l'Exemple 4 et pBF 172 préparé comme décrit dans l'Exemple 5.

11. Plasmides suivant les revendications 1 à 10, ayant comme autre particularité qu'ils provoquent dans des souches de E. coli, de préférence du sous-groupe E. coli K12, la formation de la protéine précurseur de rscu-PA avec un taux d'expression d'au moins 10 % en poids, de préférence d'au moins 14 % en poids de la protéine totale formée.

12. Plasmides suivant les revendications 1 à 11, ayant comme autre particularité qu'ils provoquent dans des souches de E. coli, de préférence du sous-groupe E. coli K12, la formation de la protéine précurseur de rscu-PA avec un taux d'expression de 10 à 25 % en poids, notamment de 14 à 20 % en poids de la protéine totale formée.

13. Procédé de production de plasmides suivant la revendication 1, caractérisé en ce qu'on insère dans le plasmide pBR 322, duquel la région nic/bom et/ou au moins une partie du gène de résistance à la tétracycline ont été enlevées, entre les coupures Eco RI et Hind III, un "site de multiclonage" présentant la séquence de nucléotides indiquée sur la figure 2, qui contient entre les coupures Xba I et Nde I la séquence de Shine-Dalgarno et, à une distance de 6 à 12, de préférence de 8 à 10 nucléotides, le codon d'initiation ATG et on incorpore avec son aide, d'une manière connue, un ou deux terminateurs de transcription, les séquences synthétiques partielles du gène de structure de scu-PA, de préférence à partir de l'extrémité terminale 3'C du gène de structure, ainsi qu'un promoteur réglable.

14. Procédé de production de plasmides suivant la revendication 1, caractérisé en ce qu'on introduit d'une manière connue le fragment Eco RI x Hind III d'un plasmide obtenu conformément à la revendication 13 comme cassette d'expression dans un autre plasmide capable de multiplication autonome dans des entérobactériacées, de préférence dans E. coli.

15. Procédé de production de plasmides suivant les revendications 1 et 2, caractérisé en ce qu'on coupe un plasmide obtenu conformément à la revendication 13 ou 14 avec Nde I, on comble les sites de coupure résultants puis on lie les extrémités franches qui ont été formées.

16. Utilisation d'un plasmide suivant les revendications 1 à 12 dans l'obtention d'un activateur de plasminogène, caractérisée en ce qu'on transforme avec le plasmide d'une manière connue une souche d'entérobactériacées, de préférence une souche de E. coli, on induit l'expression du gène de structure de scu-PA, on sépare la protéine précurseur de rscu-PA formée du milieu et des cellules bactériennes lysées, on solubilise la protéine précurseur puis on la replie en rscu-PA par l'action d'un système d'oxydo-réduction.

17. Utilisation suivant la revendication 16, caractérisée en ce qu'on transforme avec le plasmide une souche de l'espèce E. coli, de préférence une souche de E. coli K12.
